(19) Europäisches Patentamt
European Patent Office
Office européen des brevets

(11) EP 2 016 932 B1

(12) **FASCICULE DE BREVET EUROPEEN**

(45) Date de publication et mention de la délivrance du brevet:
**30.04.2014 Bulletin 2014/18**

(51) Int Cl.:
***A61K 8/04*** *(2006.01)* ***A61K 8/06*** *(2006.01)*
***A61K 8/67*** *(2006.01)* ***A61Q 19/04*** *(2006.01)*

(21) Numéro de dépôt: **08158761.0**

(22) Date de dépôt: **23.06.2008**

(54) **Utilisation pour la coloration de la peau de l'acide dehydroascorbique**

Anwendung zur Hautfärbung mit Dehydroascorbinsäure

Utilisation pour la coloration de la peau de l'acide dehydroascorbique

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **09.07.2007 FR 0756353**

(43) Date de publication de la demande:
**21.01.2009 Bulletin 2009/04**

(73) Titulaire: **L'Oréal**
**75008 Paris (FR)**

(72) Inventeurs:
- **Pruche, Francis**
  **60300, Senlis (FR)**
- **Choisy, Patrick**
  **37270, Montlouis sur Loire (FR)**

(74) Mandataire: **Miszputen, Laurent**
**L'Oréal**
**D.I.P.I.**
**25-29 Quai Aulagnier**
**92600 Asnières (FR)**

(56) Documents cités:
**EP-A- 0 954 277** **EP-A- 1 270 444**
**WO-A-2005/039510** **DE-A1- 10 149 007**
**FR-A- 2 801 788**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 016 932 B1

## Description

La présente invention se rapporte à l'utilisation cosmétique de l'acide déhydroascorbique ou l'un de ses dérivés monomères, dérivés **dimères** ou isomères dans une composition comprenant un milieu physiologiquement acceptable comme agent de coloration artificielle de la peau et plus particulièrement comme agent autobronzant de la peau.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les fonctions amines libres de la peau en particulier les acides aminés, peptides et protéines de la peau, la formation de produits colorés.

A cet effet, on sait que la dihydroxyacétone, ou DHA, est un produit particulièrement intéressant qui est couramment utilisé en cosmétique comme agent de bronzage artificiel de la peau ; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. Un autre inconvénient de la DHA est sa tendance à produire des nuances jaunes qui nuit à l'obtention d'une teinte de la peau naturelle. Il existe donc une demande croissante de produits autobronzants agissant rapidement et conférant une coloration plus proche du bronzage naturel.

Ainsi, on est toujours à la recherche de nouveaux composés et de nouvelles compositions permettant de conférer artificiellement à la peau une coloration proche du bronzage naturel d'une manière simple, efficace et rapide.

On connaît dans la demande WO2005/039510 l'utilisation de l'acide dehydroascorbique ou l'un de ses sels produit in situ par oxydation enzymatique comme agent fixateur dans la permanente des cheveux. On connaît également dans la demande de brevet DE19745354 l'utilisation de l'acide déhydroascorbique en association avec des composés avec des groupes amines primaires ou secondaires ou des groupes hydroxy particuliers pour la coloration des cheveux.

Or, à la suite d'importantes recherches menées dans le domaine de la coloration artificielle de la peau, la Demanderesse a maintenant découvert que l'acide déhydroascorbique ou l'un de ses dérivés monomères, dérivés **dimères** ou isomères permettaient de conférer, après application du produit sur la peau, une coloration artificielle proche du bronzage naturel, qui se développe plus rapidement que les autobronzants classiques comme la DHA (visible à partir de 30 minutes) et donne des couleurs plus intenses dont la composante rouge est plus marquée et permettant d'offrir selon la quantité utilisée une gamme de nuances plus proches de la pigmentation naturelle contrairement aux autobronzants classsiques comme la DHA. Les produits contenant l'acide déhydroascorbique ou l'un de ses polymères présentent aussi l'avantage d'être légèrement colorés à l'application sur la peau et de pouvoir ainsi doser et visualiser la zone d'application du produit.

La présente invention a donc pour objet l'utilisation cosmétique de l'acide déhydroascorbique ou l'un de ses dérivés monomères, dérivés **dimères** ou isomères dans une composition comprenant un milieu physiologiquement acceptable, comme agent de coloration artificielle de la peau et plus particulièrement comme agent autobronzant de la peau.

La présente invention a également pour objet un procédé de coloration artificielle de la peau et plus particulièrement un procédé de bronzage artificiel de la peau, caractérisé en ce qu'il consiste à appliquer sur celle-ci une quantité efficace d'une composition cosmétique comprenant dans un milieu physiquement acceptable au moins l'acide déhydroascorbique et/ou l'un de ses dérivés monomères répondant à la formule (I) définie ci-après et/ou l'un de leurs monomères de formule (I') définie ci-après et/ou l'un de ses dérivés **dimères** ceux de formule (II).

Au sens de la présente invention, on entendra, par « coloration artificielle de la peau », une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

Au sens de la présente invention, on entendra, par « polymère » comme molécule ayant dans sa structure au moins deux motifs récurrents.

Au sens de la présente invention, on entendra, par « milieu physiologiquement acceptable » on entend support compatible avec la peau, les ongles, les lèvres qui présente une couleur, une odeur et un toucher agréables et qui ne génère pas d'inconforts inacceptables (picotements, tiraillements, rougeurs), susceptibles de détourner la consommatrice d'utiliser cette composition comprenant un tel support.

Au sens de la présente invention, on entendra, par « agent autobronzant de la peau » un composé susceptible

de produire au contact de celle-ci une réaction colorée avec les fonctions amines libres présentes dans la peau tels que les acides aminés, les peptides ou les protéines.

D'autres caractéristiques, aspects et avantages de la présente invention apparaîtront à la lecture de la description détaillée qui va suivre.

L'acide déhydroascorbique et les dérivés monomères conformes à l'invention répondent à la formule (I) suivante ou répondent à leur forme isomère de formule (I') suivante :

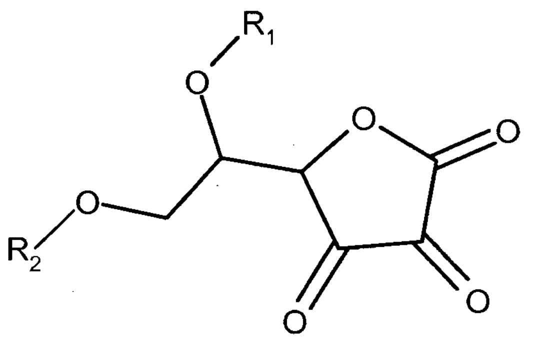

(I)

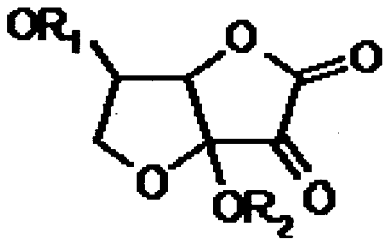

(I')

dans lesquelles $OR_1$, $OR_2$, identiques ou différents, désignent OH; un radical alcoxy en $C_1$-$C_{30}$ et plus préférentiellement en $C_1$-$C_{18}$, linéaire ou ramifié, saturé ou insaturé ; un glycoside et plus préférentiellement glucose ; un ester d'un acide carboxylique aliphatique en $C_1$-$C_{30}$ (de préference en $C_1$-$C_{18}$), linéaire ou ramifié, saturé ou insaturé, pouvant être sustitué par un groupe aryle ou un hétérocycle; un ester d'acide carboxylique arylique, hétérocyclique pouvant être substitué par au moins un radical alkyle en $C_1$-$C_{30}$ ( plus préférentiellement en $C_1$-$C_{18}$), linéaire ou ramifié, saturé ou insaturé ; un groupe phosphate ; un groupe sulfate.

De manière préférentielle, $R_2$ désignera un ester d'un acide carboxylique aliphatique en $C_1$-$C_{30}$ (de préférence en $C_1$-$C_{18}$), linéaire ou ramifié, saturé ou insaturé, pouvant être sustitué par un groupe aryle ou un hétérocycle ; un ester d'acide carboxylique arylique, hétérocyclique pouvant être substitué par au moins un radical alkyle en $C_1$-$C_{30}$ ( plus préférentiellement en $C_1$-$C_{18}$), linéaire ou ramifié, saturé ou insaturé.

L'acide dehydroascorbique de formule (I) est également appelé thréo-2,3-hexodiulsono-1,4 lactone, 9Cl, (cas 490-83-5) et a pour structure :

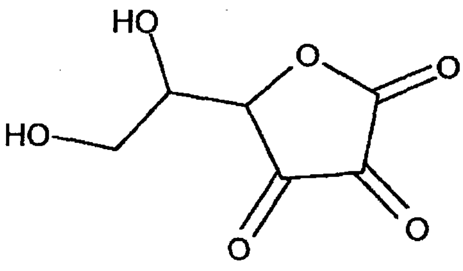

Sa forme isomère de formule (I') appelée 3a,6-dihydroxy-tetrahydro-furo[3,2-b]furan-2,3-dione a pour structure :

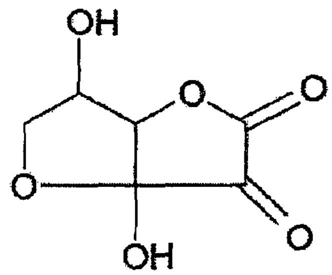

Parmi les dérivés monomères de l'acide dehydroascorbique de formule (I) et (I'), on peut citer, en particulier, les composés particuliers suivants :

- L-threo-2,3-Hexodiulosonic acid, γ-lactone, 5,6-bis-(3-phenyl-2-propenoate) (Cas # 106406-96-6) avec $OR_1$= $OR_2$ = 3-phenyl-2-propenoate
- L-threo-2,3-Hexodiulosonic acid, γ-lactone, 6-acetate (Cas # 106227-02-5) avec $OR_2$= acetate et $R_1$ = H
- L-threo-2,3-Hexodiulosonic acid, γ-lactone, 5,6-di-acetate (Cas # 59681-41-3) avec $OR_1$=$OR_2$= acetate
- L-threo-2,3-Hexodiulosonic acid, γ-lactone, 6-hexadecanoate Cas # 63247-05-2 avec $OR_2$= hexadecanoate et $OR_1$ = H
- L-threo-2,3-Hexodiulosonic acid, γ-lactone, 6-octadecanoate (9CI) (Cas # 59681-40-2) avc $OR_2$=octadecanoate et $OR_1$ = OH
- L-threo-2,3-Hexodiulosonic acid, γ-lactone, 6-benzoate (Cas # 63247-04-1) avec $OR_2$ = benzoate et $OR_1$ = OH

Parmi les exemples de dérivés **dimères** on peut citer les composé dimères de formule (II) suivante :

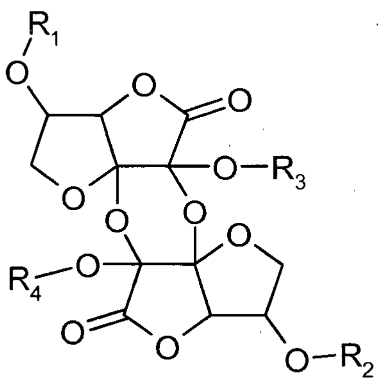

(II)

dans laquelle $OR_1$, $OR_2$, $OR_3$, $OR_4$, identiques ou différents, identiques ou différents, désignent OH ; un radical alcoxy en $C_1$-$C_{30}$ et plus préférentiellement en $C_1$-$C_{18}$, linéaire ou ramifié, saturé ou insaturé ; un glycoside et plus préférentiellement glucose ; un ester d'un acide carboxylique aliphatique en $C_1$-$C_{30}$ (de préférence en $C_1$-$C_{18}$), linéaire ou ramifié, saturé ou insaturé, pouvant être sustitué par un groupe aryle ou un hétérocycle ; un ester d'acide carboxylique arylique, hétérocyclique pouvant être substitué par au moins un radical alkyle en $C_1$-$C_{30}$ ( plus préférentiellement en $C_1$-$C_{18}$), linéaire ou ramifié, saturé ou insaturé ; un groupe phosphate ; un groupe sulfate.

De manière préférentielle, $R_1$ et/ou $R_2$ désigneront un ester d'un acide carboxylique aliphatique en $C_1$-$C_{30}$ (de préférence en $C_1$-$C_{18}$), linéaire ou ramifié, saturé ou insaturé, pouvant être sustitué par un groupe aryle ou un hétérocycle ; un ester d'acide carboxylique arylique, hétérocyclique pouvant être substitué par au moins un radical alkyle en $C_1$-$C_{30}$ ( plus préférentiellement en $C_1$-$C_{18}$), linéaire ou ramifié, saturé ou insaturé.

Parmi les dimères dérivés de l'acide dehydroascorbique de formule (II), on peut citer, en particulier, les composés particuliers suivants :

2H,8H-Bisfuro[3',2':2,3]furo[3,4-b:3',4'-e][1,4]dioxin-5,11 (5aH,11aH)-dione, 3,5a,9,11a-tetrakis (benzoyloxy)tetrahydro-[3S-(3α,3aα,5aβ, 6aS* ,9aα, 11aβ, 12aS*)] (Cas # 103559-39-3) avec OR1=OR2=OR3=OR4 = benzoate

1,6,9,13-tetraoxadispiro[4.2.4.2]tetradecane-7,14-dicarboxylic acid, 4,12-dihydroxy-3,7,11,14-tetramethoxy-, di-γ-lactone Cas # 94329-25-6 avec $R_1$=$R_2$=$R_3$=$R_4$ = methyle.

2H,8H-Bisfuro[3',2':2,3]furo[3,4-b:3',4'-e][1,4]dioxin-5,11 (5aH,11aH)-dione, 3,5a,9,11a-tetrakis (acetyloxy)tetrahydro-[3S-(3α,3aα,5aβ, 6aS*,9α, 9aα, 11aβ, 12aS*)] (Cas # 25726-18-5) avec $OR_1$=$OR_2$=$OR_3$=$OR_4$ = acetate

2H,8H-Bisfuro[3',2':2,3]furo[3,4-b:3',4'-e][1,4]dioxin-5,11 (5aH,11aH)-dione, 3,5a,9,11a-tetrakis (3"-phenyl-2"-propenoxy)tetrahydro-[3S-(3α,3aα,5aβ, 6aS*,9α, 9aα, 11aβ, 12aS*)] Cas # 106406-97-7) avec $OR_1$=$OR_2$=$OR_3$=$OR_4$= 3-phenyl-2-propenoate.

On citera plus particulièrement le composé dimère (cas 72691-25-29) avec $R_1$=$R_2$ = H de structure suivante :

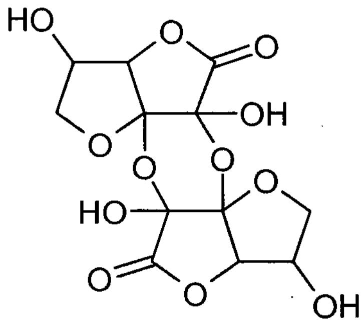

Selon une forme particulière de l'invention, l'acide déhydroascorbique ou l'un de ses dérivés monomères, polymères ou isomères peut être formé « in situ » à partir de l'acide ascorbique ou l'un de ses dérivés ou l'un de ses sels par oxydation chimique et/ou par oxydation enzymatique selon le schéma réactionnel suivant :

2 (acide ascorbique) + $O_2$ →2 (Acide déhydroascorbique) + 2 $H_2O$

La réaction d'oxydation peut être en particulier catalysée efficacement par de nombreux types d'enzymes comme par exemple les ascorbates oxydases produites par la plupart des plantes ainsi que certaines bactéries, levures ou animaux (E.C [1.10.3.3] Lee, M.H. ; Dawson , C.R. Methods Enzymol., 1979, 62, 30-39).

Un autre objet de l'invention est l'utilisation cosmétique d'un agent à deux composants comprenant

a) un premier composant (B) contenant dans un milieu physiologiquement acceptable au moins l'acide ascorbique ou l'un de ses dérivés ou sels ;
b) un deuxième composant (C) contenant dans un milieu physiologiquement acceptable au moins un agent oxydant chimique et/ou un agent oxydant enzymatique ; les composants (B) et (C) étant stockés séparément, pour la coloration artificielle de la peau en particulier comme agent autobronzant de la peau.

L'invention a également pour objet un procédé de coloration artificielle de la peau, et plus particulièrement de bronzage artificiel de la peau, caractérisé en ce qu'il consiste à appliquer sur celle-ci

a) un premier composant (B) contenant dans un milieu physiologiquement acceptable au moins l'acide ascorbique ou l'un de ses dérivés ou sels ;
b) un deuxième composant (C) contenant dans un milieu physiologiquement acceptable moins un agent oxydant chimique et/ou un agent oxydant enzymatique ; les composants (B) et (C) étant mélangés au moment de l'emploi et appliqués simultanément sur la peau ou bien appliqués sur la peau l'un après l'autre.

Les agents oxydants chimiques classiquement utilisés pour l'oxydation de l'acide ascorbique ou de ses dérivés ou sels sont par exemple le peroxyde d'hydrogène, le peroxyde d'urée, les bromates de métaux alcalins, les persels tels que les perborates et persulfates, les peracides ou leurs mélanges.

Les systèmes oxydants enzymatiques utilisés sont classiquement les enzymes oxydases utilisant soit l'oxygène de l'air soit un substrat pour former le peroxyde d'hydrogène parmi lesquelles on peut citer les oxydo-réductases à 2

électrons telles que les uricases, les ascorbate oxidases..

Les enzymes utilisées conformément à la présente invention sont de préférence choisies parmi les ascorbates oxydases utilisant l'oxygène de l'air. Plus préférentiellement, les enzymes sont choisies parmi celles appartenant à la classification selon la Commission des Enzyme [1.10.3.3].

L'enzyme ascorbate oxydase peut être par exemple dérivée des genres botaniques suivantes : *Arabidopsis Brassica, Cucumis Curcubita, Myrothecium, Nicotiana, Oryza, Titicum.* Plus préférentiellement, elle est choisie parmi celles dérivées de *Curbita pepo mudullosa* (zucchini). On peut également utiliser une enzyme ascorbate oxydase obtenues à partir de nombreux autres plantes comprenant le chou *(Brassica oleracea),* le concombre *(Cucumis savitus),* la citrouille *(Curcubita cv. Ebisu Nankin),* le tabac *(Nicotania tabacum),* la moutarde *(Sinapsis alba),* le riz *(Oryza sativa)* et le blé *(Triticum aestivum).* D'autres sources comprennent les champignons *(Myrotectium verrucaria)* et les bactéries thermophiles *(Acremonium sp. HI-25).*

L'enzyme peut être présente comme solution ou sous forme de poudre et peut être de préférence stabilisée par des tampons, de la glycérine, des sucres ou d'autres composés polyhydroxylés, des agents chélateurs de métaux comme l'EDTA, les thiols comme le thioglycérol, le mercaptoéthanol ou dithiothréitol, le polyéthylèneglycol, les protéines non-réactives ou autres conservateurs d'enzyme. On peut également stabiliser les enzymes par modification covalente selon des techniques classiques. L'enzyme peut être également immobilisée par liaison covalente sur un support solide comme des microparticules de silice modifiée en surface, d'alumine, de verre, de polyméthacrylate modifié par de l'oxyrane, de carboxyalkylcellulose, d'aminoalkylsilice, de verre aminoalkylé, d'aminoalkylcellulose. Les enzymes peuvent être également adsorbées sur des surfaces de particules modifiées hydrophobiquement ou ioniquement telles que des carboxyalkylcelluloses ou dialkylaminocelluloses. Une autre possibilité consiste en une liason covalente de l'enzyme avec un polymère hydrosoluble synthétique ou biosynthétique tels que les polyéthylèneglycols, les poly(acide acrylique), les poly(alcool vinylique), les polyéthyleneimines, le dextrane et les protéines comme la gélatine ou l'uricase.

Ladite enzyme est de préférence présente dans la composition résultant du mélange des composants (B) et (C) à des teneurs allant de 1 à 10.000 ppm, de préférence 100 à 1000 ppm.

Selon une forme particulière de l'invention, afin d'augmenter la rémanence de la couleur et/ou l'uniformisation de la couleur obtenue après application sur la peau de l'acide déshydroascorbique ou l'un de ses dérivés monomères, dérivés **dimères** ou isomères, on associe à celui-ci l'acide ascorbique ou l'un de ses dérivés ou l'un de ses sels.

Un autre objet de l'invention consiste donc en l'utilisation cosmétique comme agent de coloration artificielle de la peau et plus particulièrement comme agent autobronzant de la peau d'un produit à trois composants comprenant :

a) un premier composant (A) contenant dans un milieu physiologiquement acceptable au moins l'acide déhydroascorbique ou l'un de ses dérivés momomères, dérivés **dimères** ou isomères ;
b) un deuxième composant (B) contenant dans un milieu physiologiquement acceptable au moins l'acide ascorbique ou l'un de ses dérivés ou sels;
c) un troisième composant (C) contenant dans un milieu physiologiquement acceptable au moins un agent oxydant chimique et/ou un agent oxydant enzymatique ; les composants (A) et (B) pouvant être dans la même composition ou stockés séparément et le composant (C) étant stocké séparément de (A) et de (B).

L'invention a également pour objet un procédé de coloration artificielle de la peau et plus particulièrement de bronzage artificiel de la peau, caractérisé en ce qu'il consiste à appliquer sur celle-ci :

a) un premier composant (A) contenant dans un milieu physiologiquement acceptable au moins l'acide déhydroascorbique ou l'un de ses dérivés monomères, dérivés **dimères** ou isomères ;
b) un deuxième composant (B) contenant dans un milieu physiologiquement acceptable au moins l'acide ascorbique ou l'un de ses dérivés ou sels ;
c) un troisième composant (C) contenant dans un milieu physiologiquement acceptable au moins un agent oxydant chimique et/ou un agent oxydant enzymatique ; les composants (A), (B) et (C) étant mélangés au moment de l'emploi et appliqués sur la peau ou bien appliqués sur la peau l'un après l'autre.

L'acide déhydroascorbique ou l'un de ses dérivés monomères, dérivés **dimères** ou isomères est de préférence utilisé à des teneurs allant de 0,1 à 50% en poids et plus préférentiellement de 1 à 10% en poids par rapport au poids total de la composition le contenant.

L'acide ascorbique ou l'un de ses dérivés ou sels est de préférence utilisé à des teneurs allant de 0,1 à 50% en poids et plus préférentiellement de 1 à 15% en poids par rapport au poids total de la composition le contenant.

Les compositions de l'invention peuvent se présenter sous toutes les formes appropriées pour une application topique, notamment sous forme de gels aqueux, sous forme d'émulsions obtenues par dispersion d'une phase grasse (appelée aussi phase huileuse) dans une phase aqueuse (H/E) ou inversement (E/H) ou d'émulsions multiples (par exemple E/H/E ou H/E/H ou H/H/E). Elles peuvent être plus ou moins fluides et avoir l'aspect d'une crème blanche ou

colorée, d'une pommade, d'un lait, d'une lotion, d'un sérum, d'une pâte, de poudre, de bâtonnet solide et éventuellement être conditionnée en aérosol et se présenter sous forme de mousse ou de spray. Ces compositions sont préparées selon les méthodes usuelles.

Selon un mode particulier de réalisation de l'invention, les compositions de l'invention peuvent se présenter sous forme d'une émulsion et comporter alors au moins une phase huileuse. La proportion de la phase huileuse de l'émulsion peut aller de 1 à 80 % en poids, de préférence de 2 à 50 % en poids et mieux de 2 à 40 % en poids par rapport au poids total de la composition. Les corps gras de la phase huileuse, notamment les huiles, et les émulsionnants et co-émulsionnants éventuellement présents, utilisés dans la composition sous forme d'émulsion sont choisis parmi ceux classiquement utilisés dans le domaine cosmétique ou dermatologique. L'émulsionnant et le co-émulsionnant, quand ils sont présents, le sont généralement, en une proportion allant de 0,1 à 30 % en poids, de préférence de 0,3 à 20 % en poids et mieux de 0,5 à 15 % en poids par rapport au poids de la composition. L'émulsion peut en outre, contenir des vésicules lipidiques en plus ou à la place des émulsionnants et/ou co-émulsionnants.

Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant la phase continue de l'émulsion à obtenir (E/H ou H/E). Quand l'émulsion est multiple, elle comporte généralement un émulsionnant dans l'émulsion primaire et un émulsionnant dans la phase externe dans laquelle est introduite l'émulsion primaire.

Comme émulsionnants utilisables pour la préparation des émulsions E/H, on peut citer par exemple les alkyl esters ou éthers de sorbitan, de glycérol ou de sucres ; les tensioactifs siliconés comme les dimethicone copolyols tels que le mélange de cyclomethicone et de dimethicone copolyol, vendu sous les dénominations DC 5225 C et DC 3225 C par la société Dow Corning, et comme les alkyl-dimethicone copolyols tels que le Laurylmethicone copolyol vendu sous la dénomination "Dow Corning 5200 Formulation Aid" par la société Dow Corning, le Cetyl dimethicone copolyol vendu sous la dénomination Abil EM 90R par la société Goldschmidt et le mélange de Polyglyceryl-4 isostearate/Cetyl dimethicone copolyol/Hexyl laurate vendu sous la dénomination Abil WE 09R par la société Goldschmidt. On peut y ajouter aussi un ou plusieurs co-émulsionnants, qui, de manière avantageuse, peuvent être choisis dans le groupe comprenant les esters d'acide gras à chaîne ramifiée et de polyol, et notamment les esters d'acide gras à chaîne ramifiée et de glycérol et/ou de sorbitan et par exemple l'isostéarate de polyglycéryle, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Comme émulsionnants utilisables pour la préparation des émulsions H/E, on peut citer par exemple les émulsionnants non ioniques tels que les esters d'acides gras et de polyols oxyalkylénés (plus particulièrement polyoxyéthylénés), et par exemple les stéarates de polyéthylène glycol comme le stéarate de PEG-100, le stéarate de PEG-50 et le stéarate de PEG-40 ; les esters d'acides gras et de sorbitan oxyalkylénés comprenant par exemple de 20 à 100 OE, et par exemple ceux commercialisés sous les dénominations commerciales Tween 20 ou Tween 60 par la société Uniqema ; les éthers d'alcools gras oxyalkylénés (oxyéthylénés et/ou oxypropylénés) ; les esters de sucres, alcoxylés ou non, comme le stéarate de sucrose et comme le PEG-20 méthylglucose sesquistéarate ; les esters de sorbitan tels que le palmitate de sorbitan commercialisé sous la dénomination Span 40 par la société Uniqema ; les esters de diacide et d'alcool gras, tels que le tartrate de dimyristyle ; les mélanges de ces émulsionnants comme par exemple le mélange de stéarate de glycéryle et de stéarate de PEG-100 (nom CTFA: Glyceryl Stearate / PEG-100 Stearate) commercialisé sous la dénomination Arlacel 165 par la société Uniqema et sous la dénomination SIMULSOL 165 par la société SEPPIC ; ou le mélange de tartrate de dimyristyle, d'alcool cétéarylique, de Pareth-7 et de PEG-25 laureth-25, commercialisé sous la dénomination Cosmacol PSE par la société Sasol (nom CTFA : Dimyristyl tartrate / cetearyl alcool /12-15 Pareth 7 / PPG 25 laureth 25).

On peut ajouter à ces émulsionnants, des co-émulsionnants tels que par exemple les alcools gras ayant de 8 à 26 atomes de carbone, comme l'alcool cétylique, l'alcool stéarylique et leur mélange (alcool cétéarylique), l'octyl dodécanol, le 2-butyloctanol, le 2-hexyldécanol, le 2-undécylpentadécanol ou l'alcool oléique, ou les acides gras.

On peut aussi préparer des émulsions sans tensioactifs émulsionnants ou en contenant moins de 0,5 % du poids total de la composition (A) ou (B), en utilisant des composés appropriés, pour stabiliser lesdites émulsions par exemple des polymères amphiphiles, des électrolytes.

Quand la composition de l'invention est sous forme d'émulsion, elle comporte au moins une phase huileuse qui contient au moins une huile, notamment une huile cosmétique. On entend par "huile" un corps gras liquide à la température ambiante (25°C).

Comme huiles utilisables dans la composition de l'invention, on peut utiliser par exemple les huiles hydrocarbonées d'origine animale, telles que le perhydrosqualène (ou squalane) ; les huiles hydrocarbonées d'origine végétale, telles que les triglycérides des acides caprylique/caprique comme ceux vendus par la société Stearineries Dubois ou ceux vendus sous les dénominations Miglyol 810, 812 et 818 par la société Dynamit Nobel ou encore les huiles d'origine végétale, par exemple les huiles de tournesol, de maïs, de soja, de courge, de pépins de raisin, de sésame, de noisette,

d'abricot, de macadamia, d'arara, de coriandre, de ricin, d'avocat, l'huile de jojoba, l'huile de beurre de karité ; les huiles de synthèse ; les huiles de silicone comme les polyméthylsiloxanes (PDMS) volatiles ou non à chaîne siliconée linéaire ou cyclique, liquides ou pâteux à température ambiante ; les huiles fluorées telles que celles partiellement hydrocarbonées et/ou siliconées comme celles décrites dans le document JP-A-2-295912 ; les éthers tels que l'éther dicaprylique (nom CTFA : Dicaprylyl ether) ; et les benzoates d'alcools gras en C12-C15 (Finsolv TN de FINETEX) ; les dérivés arylalkyl benzoates comme le 2-phenylethyl benzoate (X-Tend 226 de ISP) ; les huiles amidées comme le N-lauroylsarcosinate d'isopropyle ( ELDEW SL-205 de Ajimoto) et leurs mélanges.

La composition de l'invention peut également contenir un ou plusieurs solvants organiques qui peuvent être choisis dans le groupe constitué par les solvants organiques hydrophiles, les solvants organiques lipophiles, les solvants amphiphiles ou leurs mélanges.

Parmi les solvants organiques hydrophiles, on peut citer par exemple des alcools monohydriques linéaires ou ramifiés ayant de 1 à 8 atomes de carbone comme l'éthanol, le propanol, le butanol, l'isopropanol, l'isobutanol ; des polyéthylène glycols ayant de 6 à 80 oxydes d'éthylène ; des polyols tels que le propylène glycol, l'isoprène glycol, le butylène glycol, le glycérol, le sorbitol ; les mono- ou dialkyle d'isosorbide dont les groupements alkyle ont de 1 à 5 atomes de carbone comme le diméthyl isosorbide ; les éthers de glycol comme le diéthylène glycol mono-méthyl ou mono-éthyl éther et les éthers de propylène glycol comme le dipropylène glycol méthyl éther.

Comme solvants organiques amphiphiles, on peut citer les dérivés de polypropylène glycol (PPG) tels que les esters de polypropylène glycol et d'acide gras, de PPG et d'alcool gras comme le PPG-23 oléyl éther et le PPG-36 oléate.

Comme solvants organiques lipophiles, on peut citer par exemple les esters gras tels que l'adipate de diisopropyle, l'adipate de dioctyle, les benzoates d'alkyle.

Les compositions conformes à la présente invention peuvent également comprendre des adjuvants cosmétiques classiques choisis parmi les adoucissants, les humectants, les opacifiants, les stabilisants, les émollients, les silicones, les agents anti-mousse, les parfums, les conservateurs, les tensioactifs anioniques, cationiques, non-ioniques, zwitterioniques ou amphotères, les charges, les polymères, les propulseurs, les agents alcalinisants ou acidifiants ou tout autre ingrédient habituellement utilisé dans le domaine cosmétique et/ou dermatologique.

Comme épaississants hydrophiles, on peut citer les polymères carboxyvinyliques tels que les carbopols (carbomers), les Pemulen (Copolymère acrylate/C10-C30-alkylacrylate) et les homo- et copolymères d'acrylamide et/ou d'acide 2-acrylamido-2-méthylpropane sulfonique (AMPS) comme le Sodium PolyacryloyldimthylTaurate (and) Polysorbate 80 (and) Sorbitan Oleate vendu sous le nom commercial SIMULGEL 800 par la Société SEPPIC; les dérivés cellulosiques tels que l'hydroxyéthylcellulose; les polysaccharides et notamment les gommes telles que la gomme de xanthane ; et leurs mélanges.

Comme épaississants lipophiles, on peut citer les argiles modifiées telles que le l'hectorite et ses dérivés, comme les produits commercialisés sous les noms de Bentone.

Comme conservateurs, on peut citer les esters de l'acide parahydroxybenzoïque encore appelés Parabens® (en particulier le méthyl parabène, l'éthyl parabène, le propyl parabène), le phénoxyéthanol, les libérateurs de formol comme par exemple l'imidazolidinyl urée ou la diazolidinyl urée, le digluconate de chlorhexidine, le benzoate de sodium, le caprylyl glycol, l'iodo propynyl butyl carbamate, le pentylène glycol, le bromure d'alkyl triméthylammonium tel que le bromure de myristyl-triméthylammonium (nom CTFA : bromure de Myrtrimonium), le bromure de dodécyl-triméthylammonium, le bromure d'hexadécyltriméthylammonium, et leurs mélanges tel que le mélange vendu sous la dénomination Cetrimide® par la société FEF CHEMICALS. Le conservateur peut être présent dans la composition selon l'invention en une teneur allant de 0,001 à 10 % en poids, par rapport au poids total de la composition, notamment allant de 0,1 à 5 % en poids, et en particulier allant de 0,2 à 3 % en poids.

Selon une forme particulière de l'invention, afin d'améliorer la stabilité de l'acide déhydroascorbique ou l'un de ses dérivés monomères, dérivés polymères ou isomères et /ou celle de l'acide ascorbique ou l'un de ses sels ou dérivés, chacun de ces actifs peut être encapsulé selon les techniques classiques d'encapsulation.

Les procédés de coloration artificielle de la peau et plus particulièrement de bronzage artificiel de la peau peuvent s'appliquer aux différents types de peaux.

Selon cette échelle, les différentes peaux existantes peuvent être distinguées selon les types suivants :

| Type | Réactivité de la peau | Origine |
|---|---|---|
| I | Brûle toujours, ne bronze jamais | Celte |
| II | Brûle toujours, bronze peu | Germanique |
| III | Brûle modérément, bronze progressivement | Européen |
| IV | Brûle faiblement, bronze très facilement | Méditerranéen |
| V | Brûle rarement, bronze intensément | Moyen Orient - Sud Américain |
| VI | Ne brûle jamais, fortement pigmentée | Africain |

Selon une forme particulèrement préférée de l'invention, la composition comprenant l'acide déhydroascorbique ou l'un de ses dérivés monomères de formule (I) et/ou l'un de ses isomères de formule (I') et/ou l'un de ses dérivés **dimères de formule (II)** et /ou la composition comprenant l'acide ascorbique ou l'un de ses sels ou dérivés contiennent en plus au moins un agent stabilisant et/ou au moins un agent photoprotecteur et/ou au moins un agent mouillant et/ou au moins un agent pénétrant et/ou au moins un colorant additionnel.
En effet, afin d'améliorer la stabilité de l'acide déhydroascorbique ou l'un de ses dérivés monomères, **dimères** ou isomères et /ou celle de l'acide ascorbique ou l'un de ses sels ou dérivés, les compositions conformes à l'invention les contenant peuvent inclure en plus un ou plusieurs stabilisants.
A titre d'exemple de stabilisant, on peut citer

(1) les antioxydants
(2) les chelatants,
(3) les polymères ou copolymères de N-vinylimidazole non réticulés tels que ceux décrits dans la demande de brevet EP1316302.

Selon l'invention, par polymère ou copolymère de N-vinylimidazole non réticulé on entend tout polymère comportant des unités N-vinylimidazole, et ne comportant pas d'agent réticulant. Des copolymères convenant à la mise en oeuvre de l'invention sont par exemple les copolymères comprenant des unités N-vinylimidazoles et des unités N-vinylpyrrolidone et/ou N-vinylcaprolactam.
Dans un aspect avantageux de l'invention, le copolymère possède une fraction molaire en unité N-vinylimidazole comprise entre 0,1 et 1, et plus préférentiellement entre 0,4 et 0,9.
Selon un aspect avantageux de l'invention le rapport molaire entre l'équivalent unité N-vinylimidazole et l'actif hydrophile sensible à l'oxydation varie entre 0,004 et 16 et préférentiellement entre 0,01 et 1.
De façon préférentielle on utilisera un copolymère de N-vinylimidazole / N-vinylpyrrolidone.
La masse molaire moyenne en poids des polymères de N-vinylimidazole sera avantageusement comprise entre 1000 et 1 x 107 et de préférence entre 5000 et 5 x 106.
On pourra utiliser à cet effet, le copolymère vinylpyrrolidone/vinylimidazole (50/50) ayant une masse molaire moyenne en poids de 1 200 000 vendu sous la référence LUVITEC VPI 55K72W par la société BASF ou le copolymère vinylpyrrolidone/vinylimidazole (50/50) ayant une masse molaire moyenne en poids de 10 000 vendu sous la référence LUVITEC VPI 55K18P par la société BASF. Les polymères ou copolymères selon l'invention peuvent par exemple être préparés selon la méthode décrite dans la demande de brevet WO-97/45517.

(4) les polymère amphiphiles choisis parmi les oligomères ou polymères dérivés de polyisobutylène comportant une partie apolaire polyisobutylène comprenant au moins 40 atomes de carbone et au moins une partie polaire terminale constituée d'acides ou de diacides carboxyliques, de leurs anhydrides ou de leurs formes modifiées sous forme d'esters, d'amides ou de sels, et leurs mélanges tels que décrits dans dans la demande de brevet 1481677.
Ces polymères amphiphiles sont constitués d'une partie a polaire polyisobutylène et d'au moins une partie polaire. La partie a polaire polyisobutylène comprend au moins 40 atomes de carbone et de préférence de 60 à 700 atomes de carbone. Il est important que cette partie comporte au moins 40 atomes de carbone pour atteindre le but de l'invention. S'il y a moins de 40 atomes de carbone, on n'obtient pas un système bien stable.
La partie polaire de ces oligomères ou de ces polymères amphiphiles est constituée d'acides ou de diacides carboxyliques, de leurs anhydrides ou de leurs formes modifiées sous forme d'esters, d'amides ou de sels, et leurs mélanges. De préférence, la partie polaire terminale est constituée de diacides carboxyliques ou de leurs anhydrides ou de leurs formes modifiées sous forme d'esters, d'amides ou de sels.
Par formes modifiées sous forme d'esters, d'amides ou de sels, on désigne les acides ou diacides carboxyliques modifiés par des alcools, des amines, des alcanolamines ou des polyols, ou encore sous forme de sels de métal alcalin ou alcalino-terreux, d'ammonium ou encore de base organique comme les sels de diéthanolamine et de triéthanolamine.
Les oligomères ou polymères dérivés d'acide ou d'anhydride succinique peuvent être choisis notamment parmi les dérivés polyisobutylène d'acide ou d'anhydride succinique décrits dans les brevets US-A-4,234,435, US-A-4,708,753, US-A-5,129,972, US-A-4,931,110, GB-A-2,156,799 et US-A-4,919,179. La partie polyisobutylène peut être hydrogénée ou non, de poids moléculaire allant de 400 à 5000. Dans le polyisobutylène à terminaison succinique ainsi obtenu, la partie succinique peut être estérifiée, amidifiée ou sous forme de sel, c'est-à-dire qu'elle peut être avantageusement modifiée par des alcools, des amines, des alcanolamines ou des polyols, ou encore se trouver sous forme de sels de métal alcalin ou alcalino-terreux, d'ammonium ou encore de base organique comme les sels de diéthanolamine et de triéthanolamine. Les polyisobutylènes à terminaison succinique estérifiée ou amidifiée sont des produits de réaction de (a) un polyisobutylène à terminaison succinique, et de (b) une amine ou un alcool, pour former une amide ou un ester. Le terme « amine » utilisé ici comprend tous types d'amines dont les alcanolamines.

Il peut s'agir par exemple de mono-amines primaires, secondaires ou tertiaires, ces amines pouvant être aliphatiques, cycloaliphatiques, aromatiques, hétérocycliques, saturées ou insaturées. Par ailleurs, les alcools peuvent être des mono- ou poly-alcools. Les mono-alcools comprennent les alcools aliphatiques primaires, secondaires ou tertiaires, et les phénols. Les poly-alcools peuvent être par exemple choisis parmi les poly-alcools aliphatique, cycloaliphatiques, aromatiques et hétérocycliques. Les polyisobutylènes à terminaison succinique modifiée (estérifiée ou amidifiée) et leur procédé de préparation sont décrits en particulier dans le document US-A-4,708,753.

Comme polyisobutylène à terminaison succinique, on peut citer notamment les polyisobutylènes à terminaison succinique modifiée, tels que les produits commercialisés sous les dénominations LUBRIZOL 5603 et LUBRIZOL 2650 par la société Lubrizol. Selon un mode préféré de réalisation de l'invention, on utilise le polymère commercialisé sous la dénomination LUBRIZOL 5603 par la société Lubrizol, qui est le sel de diéthyléthanolamine de polyisobutylène à terminaison succinique estérifiée (nom INCI : Hydroxyethyldiethonium polyisobutenyl triethylaminosuccinate / diethylethanolamine).

Un autre exemple de dérivé de polyisobutylène utilisable dans l'invention est le produit de la réaction de l'anhydride maléique avec le polyisobutylène, tel que le produit commercialisé sous la dénomination Glissopal SA par la société BASF.

(5) les copolymères d'anhydride méléique comportant un ou plusieurs co-monomères anhydride maléique et un ou plusieurs co-monomères choisis parmi l'acétate de vinyle, l'alcool vinylique, la vinylpyrrolidone, les oléfines comportant de 2 à 20 atomes de carbones et le styrène tels que décrits dans le brevet EP1374849.

Selon l'invention, par copolymère d'anhydride maléique, on entend tout polymère obtenu par copolymérisation d'un ou plusieurs co-monomères anhydride maléique et d'un ou plusieurs co-monomères choisis parmi l'acétate de vinyle, l'alcool vinylique, la vinylpyrrolidone, les oléfines comportant de 2 à 20 atomes de carbones comme l'octadécène, l'éthylène, l'isobutylène, le diisobutylène, l'isooctylène, et le styrène, les co-monomères anhydride maléique étant optionnellement hydrolysés, partiellement ou totalement. De préférence on utilisera des polymères hydrophiles, c'est à dire des polymères ayant une solubilité dans l'eau supérieure ou égale à 2 g/l.

Des copolymères convenant plus particulièrement à la mise en oeuvre de l'invention sont des copolymères obtenu par copolymérisation d'une ou plusieurs unités anhydride maléique et dont les unités anhydride maléiques sont sous forme hydrolysée, et préférentiellement sous forme de sels alcalins, par exemple sous forme de sels d'ammonium, de sodium, de potassium ou de lithium.

Dans un aspect avantageux de l'invention, le copolymère possède une fraction molaire en unité anhydride maléique comprise entre 0,1 et 1, et plus préférentiellement entre 0,4 et 0,9.

Selon un aspect avantageux de l'invention le rapport molaire entre l'équivalent unité anhydride maléique et l'actif hydrophile sensible à l'oxydation varie entre 0,005 et 10 et préférentiellement entre 0,01 et 1.

La masse molaire moyenne en poids des copolymères d'anhydride maléique sera avantageusement comprise entre 1000 et 500 000 et de préférence entre 1000 et 50 000.

De façon préférentielle on utilisera un copolymère de styrène et d'anhydride maléique dans un rapport 50/50.

On pourra utiliser par exemple, le copolymère styrène/anhydride maléique (50/50), sous forme de sel d'ammonium à 30% dans l'eau vendu sous la référence SMA1000H® par la société ATOFINA ou le copolymère styrène/anhydride maléique (50/50), sous forme de sel de sodium à 40% dans l'eau vendu sous la référence SMA1000HNa® par la société ATOFINA.

Afin d'améliorer la photostabilité de l'acide déhydroascorbique ou l'un de ses dérivés monomères, dérivés polymères ou isomères et /ou celle de l'acide ascorbique ou l'un de ses sels ou dérivés, les compositions conformes à l'invention les contenant peuvent inclure un ou plusieurs agents photoprotecteurs.

Les agents photoprotecteurs conformes à l'invention sont choisis parmi des filtres UV organiques et/ou inorganiques actifs dans l'UVA et/ou l'UVB hydrophiles et/ou lipophiles et/ou bien insolubles dans les solvants cosmétiques couramment utilisés.

Les filtres UV organiques hydrophiles, lipophiles ou insolubles sont notamment choisis parmi les anthranilates ; les dérivés de dibenzoylméthane ; les dérivés cinnamiques ; les dérivés salicyliques, les dérivés du camphre ; les dérivés de la benzophénone ; les dérivés de $\beta,\beta$-diphénylacrylate ; les dérivés de triazine ; les dérivés de benzotriazole ; les dérivés de benzalmalonate notamment ceux cités dans le brevet US5624663, les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US 5,166,355, GB2303549, DE 197 26 184 et EP893119 ; les dérivés de benzoxazole tels que décrits dans les demandes de brevet EP0832642, EP1027883, EP1300137 et DE10162844 ; les polymères filtres et silicones filtres tels que ceux décrits notamment dans la demande WO-93/04665 ; les dimères dérivés d'$\alpha$-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

Comme exemples de filtres UV organiques, on peut citer ceux désignés ci-dessous sous leur nom INCI :

Dérivés de l'acide para-aminobenzoiaue :

PABA,
Ethyl PABA,
Ethyl Dihydroxypropyl PABA,
Ethylhexyl Diméthyl PABA vendu notamment sous le nom « ESCALOL 507 » par ISP,
Glyceryl PABA,
PEG-25 PABA vendu sous le nom « UVINUL P25 » par BASF,

Dérivés du dibenzoylméthane :

Butyl Methoxydibenzoylmethane vendu notamment sous le nom commercial « PARSOL 1789 » par HOFFMANN LAROCHE,
Isopropyl Dibenzoylmethane,

Dérivés salicyliques :

Homosalate vendu sous le nom « Eusolex HMS » par Rona/EM Industries,
Ethylhexyl Salicylate vendu sous le nom « NEO HELIOPAN OS » par Haarmann et REIMER,
Dipropyleneglycol Salicylate vendu sous le nom « DIPSAL » par SCHER,
TEA Salicylate, vendu sous le nom « NEO HELIOPAN TS » par Haarmann et REIMER,

Dérivés cinnamiques :

Ethylhexyl Methoxycinnamate vendu notamment sous le nom commercial « PARSOL MCX » par HOFFMANN LA ROCHE,
Isopropyl Methoxy cinnamate,
Isoamyl Methoxy cinnamate vendu sous le nom commercial « NEO HELIOPAN E 1000 » par HAARMANN et REIMER,
Cinoxate,
DEA Methoxycinnamate,
- Diisopropyl Methylcinnamate,
Glyceryl Ethylhexanoate Dimethoxycinnamate

Dérivés de β-β-diphénylacrylate :

Octocrylene vendu notamment sous le nom commercial « UVINUL N539 » par BASF,
Etocrylene, vendu notamment sous le nom commercial « UVINUL N35 » par BASF,

Dérivés de la benzophénone :

Benzophenone-1 vendu sous le nom commercial « UVINUL 400 » par BASF,
Benzophenone-2 vendu sous le nom commercial « UVINUL D50 » par BASF
Benzophenone-3 ou Oxybenzone, vendu sous le nom commercial « UVINUL M40 » par BASF,
Benzophenone-4 vendu sous le nom commercial « UVINUL MS40 » par BASF,
Benzophenone-5
Benzophenone-6 vendu sous le nom commercial « Helisorb 11 » par Norquay
Benzophenone-8 vendu sous le nom commercial « Spectra-Sorb UV-24 » par American Cyanamid
Benzophenone-9 vendu sous le nom commercial« UVINUL DS-49» par BASF,
Benzophenone-12
2-((4-diethylamino-2-hydroxybenzoyl)-benzoate de n-hexyle.

Dérivés du benzylidène camphre :

3-Benzylidene camphor fabriqué sous le nom « MEXORYL SD» par CHIMEX,
4-Methylbenzylidene camphor vendu sous le nom « EUSOLEX 6300 » par MERCK,
Benzylidene Camphor Sulfonic Acid fabriqué sous le nom « MEXORYL SL» par CHIMEX,
Camphor Benzalkonium Methosulfate fabriqué sous le nom « MEXORYL SO » par CHIMEX,

Terephthalylidene Dicamphor Sulfonic Acid fabriqué sous le nom « MEXORYL SX » par CHIMEX,
Polyacrylamidomethyl Benzylidene Camphor fabriqué sous le nom « MEXORYL SW » par CHIMEX,

Dérivés du phenyl benzimidazole :

Phenylbenzimidazole Sulfonic Acid vendu notamment sous le nom commercial « EUSOLEX 232 » par MERCK,
Disodium Phenyl Dibenzimidazole Tetra-sulfonate vendu sous le nom commercial commercial « NEO HELIOPAN AP » par Haarmann et REIMER,

Dérivés du phenyl benzotriazole :

Drometrizole Trisiloxane vendu sous le nom « Silatrizole » par RHODIA CHIMIE ,
Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS,

Dérivés de triazine :

Bis-Ethylhexyloxyphenol Methoxyphenyl Triazine vendu sous le nom commercial «TINOSORB S » par CIBA GEIGY,
Ethylhexyl triazone vendu notamment sous le nom commercial «UVINUL T150 » par BASF,
Diethylhexyl Butamido Triazone vendu sous le nom commercial « UVASORB HEB » par SIGMA 3V,
2,4,6-tris(4'-amino benzalmalonate de dinéopentyle)-s-triazine
les filtres triazines symétriques décrits dans le brevet US6,225,467, la demande W02004/085412 (voir composés 6 et 9) ou le document « Symetrical Triazine Derivatives » IP.COM Journal , IP.COM INC WEST HENRIETTA, NY, US (20 septembre 2004) notamment les 2,4,6-tris-(biphényl)-1,3,5-triazines (en particulier la 2,4,6-tris(biphenyl-4-yl-1,3,5-triazine) et la 2,4,6-tris(terphenyl)-1,3,5-triazine qui est repris dans les demandes de brevet WO06/035000, WO06/034982, WO06/034991, WO06/035007, WO2006/034992, WO2006/034985.

Dérivés anthraniliques :

Menthyl anthranilate vendu sous le nom commercial commercial « NEO HELIOPAN MA » par Haarmann et REIMER,

Dérivés d'imidazolines :

Ethylhexyl Dimethoxybenzylidene Dioxoimidazoline Propionate,

Dérivés du benzalmalonate :

Di-néopentyl 4'-méthoxybenzalmalonate
Polyorganosiloxane à fonctions benzalmalonate comme le Polysilicone-15 vendu sous la dénomination commerciale « PARSOL SLX » par HOFFMANN LA ROCHE

Dérivés de 4,4-diarylbutadiène :

- 1,1-dicarboxy (2,2'-diméthyl-propyl)-4,4-diphénylbutadiène

Dérivés de benzoxazole :

2,4-bis-[5-1 (diméthylpropyl)benzoxazol-2-yl-(4-phenyl)-imino]-6-(2-ethylhexyl)-imino-1,3,5-triazine vendu sous le nom d'Uvasorb K2A par Sigma 3V
et leurs mélanges.

Les filtres UV organiques préférentiels sont choisis parmi
Ethylhexyl Methoxycinnamate
Homosalate
Ethylhexyl Salicylate,

Octocrylene,
Phenylbenzimidazole Sulfonic Acid,
Benzophenone-3,
Benzophenone-4,
Benzophenone-5,
2-(4-diethylamino-2-hydroxybenzoyl)-benzoate de n-hexyle.
4-Methylbenzylidene camphor,
Terephthalylidene Dicamphor Sulfonic Acid,
Disodium Phenyl Dibenzimidazole Tetra-sulfonate,
Ethylhexyl triazone,
Bis-Ethylhexyloxyphenol Methoxyphenyl Triazine
Diethylhexyl Butamido Triazone,
2,4,6-tris(4'-amino benzalmalonate de dinéopentyle)-s-triazine
la 2,4,6-tris-(4'-amino benzalmalonate de diisobutyle)-s- triazine
la 2,4,6-tris(biphenyl-4-yl-1,3,5-triazine)
la 2,4,6-tris(terphenyl)-1,3,5-triazine
Methylène bis-Benzotriazolyl Tetramethylbutylphénol,
Drometrizole Trisiloxane
Polysilicone-15
Di-néopentyl 4'-méthoxybenzalmalonate
1,1-dicarboxy (2,2'-diméthyl-propyl)-4,4-diphénylbutadiène
2,4-bis-[5-1 (diméthylpropyl)benzoxazol-2-yl-(4-phenyl)-imino]-6-(2-ethylhexyl)-imino-1,3,5-triazine
et leurs mélanges.

Les filtres inorganiques sont choisis parmi des pigments (taille moyenne des particules primaires: généralement entre 5 nm et 100 nm, de préférence entre 10 nm et 50 nm) d'oxydes métalliques enrobés ou non comme par exemple des pigments d'oxyde de titane (amorphe ou cristallisé sous forme rutile et/ou anatase), de fer, de zinc, de zirconium ou de cérium qui sont tous des agents photoprotecteurs UV bien connus en soi.

Les pigments peuvent être enrobés ou non enrobés.

Les pigments enrobés sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p. 53-64, tels que des aminoacides, de la cire d'abeille, des acides gras, des alcools gras, des tensio-actifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

De façon connue, les silicones sont des polymères ou oligomères organo-siliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium.

Le terme "silicones" englobe également les silanes nécessaires à leur préparation, en particulier, les alkyl silanes.

Les silicones utilisées pour l'enrobage des pigments convenant à la présente invention sont de préférence choisies dans le groupe contenant les alkyl silanes, les polydialkylsiloxanes, et les polyalkylhydrogénosiloxanes. Plus préférentiellement encore, les silicones sont choisies dans le groupe contenant l'octyl triméthyl silane, les polydiméthylsiloxanes et les polyméthylhydro-génosiloxanes.

Bien entendu, les pigments d'oxydes métalliques avant leur traitement par des silicones, peuvent avoir été traités par d'autres agents de surface, en particulier par de l'oxyde de cérium, de l'alumine, de la silice, des composés de l'aluminium, des composés du silicium, ou leurs mélanges.

Les pigments enrobés sont plus particulièrement des oxydes de titane enrobés :

- de silice tels que le produit "SUNVEIL" de la société IKEDA,
- de silice et d'oxyde de fer tels que le produit "SUNVEIL F" de la société IKEDA,
- de silice et d'alumine tels que les produits "MICROTITANIUM DIOXIDE MT 500 SA" et "MICROTITANIUM DIOXIDE MT 100 SA" de la société TAYCA, "TIOVEIL" de la société TIOXIDE, et « Mirasun TiW 60 » de la société Rhodia,
- d'alumine tels que les produits "TIPAQUE TTO-55 (B)" et "TIPAQUE TTO-55 (A)" de la société ISHIHARA, et "UVT 14/4" de la société KEMIRA,
- d'alumine et de stéarate d'aluminium tels que le produit "MICROTITANIUM DIOXIDE MT 100 T, MT 100 TX, MT 100 Z, MT-01 de la société TAYCA, les produits "Solaveil CT-10 W" et "Solaveil CT 100" de la société UNIQEMA

et le produit " Eusolex T-AVO" de la société MERCK,

- de silice, d'alumine et d'acide alginique tel que le produit "MT-100 AQ" de la société TAYCA,
- d'alumine et de laurate d'aluminium tel que le produit "MICROTITANIUM DIOXIDE MT 100 S" de la société TAYCA,
- d'oxyde de fer et de stéarate de fer tels que le produit "MICROTITANIUM DIOXIDE MT 100 F" de la société TAYCA,
- d'oxyde de zinc et de stéarate de zinc tels que le produit "BR351" de la société TAYCA,
- de silice et d'alumine et traités par une silicone tels que les produits "MICROTITANIUM DIOXIDE MT 600 SAS", "MICROTITANIUM DIOXIDE MT 500 SAS" ou "MICROTITANIUM DIOXIDE MT 100 SAS"de la société TAYCA,
- de silice, d'alumine, de stéarate d'aluminium et traités par une silicone tels que le produit "STT-30-DS" de la société TITAN KOGYO,
- de silice et traité par une silicone tel que le produit "UV-TITAN X 195" de la société KEMIRA,
- d'alumine et traités par une silicone tels que les produits "TIPAQUE TTO-55 (S)" de la société ISHIHARA, ou "UV TITAN M 262" de la société KEMIRA, de triéthanolamine tels que le produit "STT-65-S" de la société TITAN KOGYO,
- d'acide stéarique tels que le produit "TIPAQUE TTO-55 (C)" de la société ISHIHARA,
- d'hexamétaphosphate de sodium tels que le produit "MICROTITANIUM DIOXIDE MT 150 W" de la société TAYCA.

D'autres pigments d'oxyde de titane traités avec une silicone sont de préférence le TiO2 traité par l'octyl triméthyl silane et dont la taille moyenne des particules élémentaires est comprise entre 25 et 40 nm tel que celui vendu sous la dénomination commerciale "T 805" par la société DEGUSSA SILICES, le TiO2 traité par un polydiméthylsiloxane et dont la taille moyenne des particules élémentaires est de 21 nm tel que celui vendu sous la dénomination commerciale "70250 Cardre UF TiO2SI3" par la société CARDRE, le TiO2 anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les pigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les pigments d'oxyde de zinc non enrobés, sont par exemple

- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les pigments d'oxyde de zinc enrobés sont par exemple

- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40% dans le Finsolv TN, benzoate d'alcools en C12-C15) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30% ou 50% de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'ethylhexyle / copolymère PVP-hexadecene / methicone) ;
- ceux commercialisés sous la dénomination "Fuji ZnO-SMS-10" par la société Fuji Pigment (ZnO enrobé silice et polymethylsilsesquioxane) ;
- ceux commercialisés sous la dénomination "Nanox Gel TN" par la société Elementis (ZnO dispersé à 55% dans du benzoate d'alcools en C12-C15 avec polycondensat d'acide hydroxystéarique).

Les pigments d'oxyde de cérium non enrobé sont vendus par exemple sous la dénomination "COLLOIDAL CERIUM OXIDE" par la société RHONE POULENC. Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les pigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les agents photoprotecteurs sont généralement présents dans les compositions contenant l'acide déhydroascorbique ou l'un de ses polymères et /ou les compositions comprenant l'acide ascorbique ou l'un de ses sels ou dérivés dans des proportions allant de 0,01 à 20% en poids par rapport au poids total de la composition, et de préférence allant de 0,1 à 10% en poids par rapport au poids total de la composition.

Afin d'augmenter la rémanence de la couleur de la peau et/ou l'homogénéité de la couleur, les compositions contenant l'acide déhydroascorbique ou l'un de ses dérivés monomères, dérivés polymères ou isomères et /ou les compositions comprenant l'acide ascorbique ou l'un de ses sels ou dérivés selon l'invention peuvent également comprendre en plus un agent mouillant et/ou pénétrant comme par exemple l'urée, l'hydroxyethylurée, les polyols comme la glycérine, les alkylèneglycols comme le propylèneglycol, le butylèneglycol, les alkylenglycolalkyléthers comme le propyleneglycol monoethylether.

Afin de nuancer la couleur obtenue par les différents procédés de coloration artificielle tels que décrits précédemment et de mieux l'adapter aux différents types de teint de peau, les compositions contenant l'acide déhydroascorbique ou l'un de ses dérivés monomères, dérivés polymères ou isomères et /ou les compositions comprenant l'acide ascorbique ou l'un de ses sels ou dérivés conformes à la présente invention peuvent également comprendre un ou plusieurs agents de coloration additionnels.

Les agents de coloration additionnels peuvent être choisis parmi les colorants directs synthétiques ou naturels. Il peut s'agir de colorants organiques ou minéraux.

Les colorants minéraux peuvent être par exemple des pigments d'oxyde de fer dont la taille moyenne des particules élémentaires est inférieure à 100 nm tels que ceux décrits dans la demande de brevet EP 966 953.

Les colorants organiques liposolubles, synthétiques ou naturels sont, par exemple, le DC Red 17, le DC Red 21, le DC Red 27, le DC Green 6, le DC

Yellow 11, le DC Violet 2, le DC Orange 5, le rouge Soudan, les carotènes (le β -carotène, le lycopène), les xanthophylles (capsanthine, capsorubine, lutéine), l'huile de palme, le brun Soudan, le jaune quinoléine, le rocou, le curcumin.

Les colorants hydrosolubles synthétiques ou naturels sont par exemple le FDC Red 4, le DC Red 6, le DC Red 22, le DC Red 28, le DC Red 30, le DC Red 33, le DC Orange 4, le DC Yellow 5, le DC Yellow 6, le DC Yellow 8, le FDC Green 3, le DC Green 5, le FDC Blue 1, la bétanine (betterave), le carmin, la chlorophylline cuivrée, le bleu de méthylène, les anthocyanines (enocianine, carotte noire, hibiscus, sureau), la riboflavine.

Les colorants peuvent encore être choisis parmi les antraquinones , le caramel, le carmin, le noir de charbon, , les bleus azulènes, le methoxalène, le trioxalène, le guajazulène, le chamuzulène, le rose de bengale, la cosine 10B, la cyanosine, la daphinine, la juglone, la lawsone, les extraits de soja fermenté, d'algues, de champignons, de microorganismes, le sels de Flavylium non substitués en position 3 tels que par exemple ceux décrits dans le brevet EP 1 172 091, les extraits de Gesneria Fulgens, Blechum Procerum, Saxifraga et les pigments susceptibles d'être obtenus par extraction par un solvant organique ou hydroorganique d'un milieu de culture de micromycètes du type monascus Monascus.

Ces colorants peuvent être également choisis parmi les dérivés indoliques comme les monohydroxyindoles tels que décrits dans le brevet FR2651126 ( ie : 4-, 5-, 6- ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole).

Les composition selon l'invention peuvent comprendre en plus des actifs additionnels notamment choisis parmi les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, les agents tenseurs, les agents liporestructurants, les agents amincissants, les agents favorisant la microcirculation cutanée, les agents apaisants et/ou anti-irritants, les sébo-régulateurs ou anti-séborrhéiques, les agents astringents, les agents cicatrisants, les agents anti-inflammatoires et les agents anti-acné.

L'homme du métier choisira le ou lesdits actifs en fonction de l'effet recherché sur la peau, les lèvres, les ongles,

les cils ou les sourcils.

La composition selon l'invention pourra comprendre en outre au moins un ingrédient additionnel destiné à compléter l'effet biologique de ces actifs ou apporter un effet immédiat visuel ; on peut citer notamment les agents matifiants, les charges à effet flouteur, les agents fluorescents, les agents favorisant la coloration naturellement rosée de la peau et les charges abrasives ou exfoliantes.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

Pour le soin des peaux âgées, il choisira de préférence au moins un actif choisi parmi les agents hydratants, les agents desquamants, les agents améliorant la fonction barrière, les agents dépigmentants, les agents antioxydants, les agents dermodécontractants, les agents anti-glycation, les agents stimulant la synthèse de macromolécules dermiques et/ou épidermiques et/ou empêchant leur dégradation, les agents stimulant la prolifération des fibroblastes ou des kératinocytes et/ou la différenciation des kératinocytes, les agents favorisant la maturation de l'enveloppe cornée, les inhibiteurs de NO-synthases, les antagonistes des récepteurs périphériques des benzodiazépines (PBR), les agents augmentant l'activité de la glande sébacée, les agents stimulant le métabolisme énergétique des cellules, , les agents liporestructurants et les agents favorisant la microcirculation cutanée pour le contour des yeux.

Pour le soin des peaux grasses, l'homme du métier choisira de préférence au moins un actif choisi parmi les agents desquamants, agents sébo-régulateurs ou anti-séborrhéiques, les agents astringents.

Pour le soin de peaux à tendance acnéique, il choisira de préférence au moins un actif choisi parmi les agents anti-acné, les agents cicatrisants, les agents anti-inflammatoires.

Pour un soin amincissant du corps, il choisira de préférence un actif choisi parmi les actifs amincissants et les actifs favorisant la microcirculation cutanée.

Des exemples de tels composés sont décrits ci-après.

## 1. Agents hydratants ou humectants

Comme agents humectants ou hydratants, on peut citer notamment le glycérol et ses dérivés, l'urée et ses dérivés notamment l'Hydrovance® commercialisée par National Starch, les acides lactiques, l'acide hyaluronique, les AHA, les BHA, le pidolate de sodium, le xylitol, la sérine, le lactate de sodium, l'ectoine et ses dérivés, le chitosane et ses dérivés, le collagène, le plancton, un extrait d'imperata cylindra commercialisé sous la dénomination Moist 24® par la société Sederma, des homopolymères d'acide acrylique comme le Lipidure-HM® de NOF corporation, le beta-glucan et en particulier le sodium carboxymethyl beta-glucane de Mibelle-AG-Biochemistry ; un mélange d'huiles de passiflore, d'abricot, maïs, et son de riz commercialisé par Nestlé sous la dénomination NutraLipids®. ; un dérivé C-glycoside tel que ceux décrits dans la demande WO 02/051828 et en particulier le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® » ; une huile de rosier muscat commercialisée par Nestlé ; un extrait de micro-algue Prophyridium cruentum enrichi en zinc commercialisé par Vincience sous la dénomination Algualane Zinc®. ; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon.

De préférence, on utilisera un agent hydratant choisi parmi l'urée et ses dérivés notamment l'Hydrovance® commercialisée par National Starch, l'acide hyaluronique, les AHA, les BHA, des homopolymères d'acide acrylique comme le Lipidure-HM® de NOF corporation, le beta-glucan et en particulier le sodium carboxymethyl beta-glucane de Mibelle-AG-Biochemistry ; un mélange d'huiles de passiflore, d'abricot, maïs, et son de riz commercialisé par Nestlé sous la dénomination NutraLipids® ; un dérivé de C-glycoside tel que ceux décrits dans la demande WO 02/051828 et en particulier le C-β-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® » ; une huile de rosier muscat commercialisée par Nestlé ; un extrait de micro-algue Prophyridium cruentum enrichi en zinc commercialisé par Vincience sous la dénomination Algualane Zinc®.; des sphères de collagène et de chondroitine sulfate d'origine marine (Ateocollagen) commercialisées par la société Engelhard Lyon sous la dénomination sphères de comblement marines ; des sphères d'acide hyaluronique telles que celles commercialisées par la société Engelhard Lyon.

## 2. Agents desquamants

Par "agent desquamant", on entend tout composé capable d'agir :

- soit directement sur la desquamation en favorisant l'exfoliation, tel que les β-hydroxyacides (BHA), en particulier

l'acide salicylique et ses dérivés (dont l'acide n-octanoyl 5-salicylique autrement nommé capryloyl salicylic acid en nom INCI), les $\alpha$-hydroxyacides (AHA), tels que les acides glycolique, citrique, lactique, tartrique, malique ou mandélique ; l'acide 8-hexadécène-1,16-dicarboxylique ou acide 9-octadécène dioïque ; l'urée et ses dérivés; l'acide gentisique et ses dérivés ; les oligofucoses ; l'acide cinnamique ; l'extrait de Saphora japonica ; le resvératrol et certains dérivés d'acide jasmonique ;

soit sur les enzymes impliquées dans la desquamation ou la dégradation des cornéodesmosomes, les glycosidases, la stratum corneum chymotryptic enzym (SCCE) voire d'autres protéases (trypsine, chymotrypsine-like). On peut citer les composés aminosulfoniques et en particulier l'acide 4-(2-hydroxyethyl)piperazine-1-propanesulfonique (HEPES) ; l'acide 2-oxothiazolidine-4-carboxylique (procystéine) et ses dérivés ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés de sucre tels que l'O-octanoyl-6-D-maltose et la N-acétyl glucosamine.

Comme autres agents desquamants utilisables dans la composition selon l'invention, on peut citer :

- les oligofructoses, l'EDTA et ses dérivés, les laminaria extract, le o-linoleyl-6D-glucose, l'acide (3-hydroxy-2pentylcyclopentyl) acétique, le trilactate de glycérol, 1'0-octanyl-6'-D-maltose, la S carboxyméthyl cystéine, les dérivés siliciés de salicylate comme ceux décrits dans le brevet EP 0 796 861, les oligofucase comme ceux décrits dans le brevet EP 0 218 200, les sels d'acide 5-acyl salicylique, des actifs ayant des effets sur la transglutaminase comme dans le brevet EP 0 899 330,
- l'extrait de fleur de ficus opuntia indica comme l'Exfolactive® de Silab,
- l'acide 8-hexadécène 1,16-dicarboxylique,
- les esters de glucose et de vitamine F, et
- leurs mélanges.

Comme agents desquamants préférés, on pourra citer les beta-hydroxyacides, tel que l'acide n-octanoyl 5-salicylique ; l'urée ; les acides glycolique, citrique, lactique, tartrique, malique ou mandélique ; l'acide 4-(2-hydroxyethyl)piperazine-1-propanesulfonique (HEPES) ; l'extrait de Saphora japonica ; le miel ; le N-acétyl glucosamine ; le méthyl glycine diacétate de sodium, et leurs mélanges.

Encore plus préférentiellement on utilisera dans les compositions de l'invention un agent edsquamant choisi parmi l'acide n-octanoyl 5-salicylique ; l'urée ; l'acide 4-(2-hydroxyethyl)piperazine-1-propanesulfonique (HEPES) ; l'extrait de Saphora japonica ; le miel ; le N-acétyl glucosamine ; le méthyl glycine diacétate de sodium, et leurs mélanges.

**3. Agents améliorant la fonction barrière**

Comme agents améliorant la fonction barrière, on peut citer notamment l'arginine, la sérine, un extrait de Thermus thermophilus tel que le Vénucéane® de Sederma, un extrait de rhizome d'igname sauvage (dioscorea villosa) tel que l'Actigen Y® d'Active Organics, des extraits de plancton comme l'omega plancton® de Secma, des extraits de levure comme le Relipidium® de Coletica, un extrait de chataigne tel que la Recoverine® de Silab, un extrait de bourgeon de cèdre tel que le Gatuline Zen® de Gattefossé, des sphingosines comme la salicyloyl sphingosine vendue sous la dénomination « Phytosphingosine® SLC » par la société Degussa, un mélange de xylitol, de xylityl polyglycoside et de xylitan comme l'Aquaxyl® de Seppic, des extraits de solanacée comme le Lipidessence® de Coletica ; les huiles insaturées en oméga 3 telles que les huiles de rosier muscat et leurs mélanges.

On peut encore citer notamment les céramides ou dérivé, en particulier les céramides de type 2 (comme la N-oléoyldihydrosphingosine ), de type 3 (comme la stearoyl-4-hydroxysphinganine en nom INCI) et de type 5 (comme la N-2-hydroxypalmitoyldihydrosphingosine, ayant pour nom INCI :hydroxypalmytoyl sphinganine), les composés à base de sphingoïdes, les glycosphingolipides, les phospholipides, le cholestérol et ses dérivés, les phytostérols, les acides gras essentiels, le diacylglycérol, la 4-chromanone et dérivés de chromone la vaseline, la lanoline, les beures de karité, le cocoa butter, la lanoline, les sels PCA.

Comme agents préférés ayant un effet restructurant de la barrière cutanée, on citera un extrait de Thermus thermophilus, un extrait de rhizome d'igname sauvage (dioscorea villosa), un extrait de levure, un extrait de chataigne, un extrait de bourgeon de cèdre, l'arginine, la sérine, les céramides notamment de type 3 et 5 ; et leurs mélanges.

De préférence, on utilisera la sérine, l'aginine ou leur mélange.

**4. Agents dépigmentants**

Comme agents dépigmentants, on pourra citer notamment l'alpha et la béta arbutine, l'acide férulique, le lucinol et ses dérivés, l'acide kojique, le résorcinol et ses dérivés, l'acide tranexamique et ses dérivés, l'acide gentisique,

l'homogentisate, le méthyl gentisate ou l'homogentisate , l'acide dioique, le D calcium panthéteine sulfonate, l'acide lipoique, l'acide ellagique, la vitamine B3, l'acide linoléique et ses dérivés, les céramides et leurs homologues, les dérivés de plantes comme la camomille, la busserole, la famille des aloe (vera, ferox, bardensis) , de murier, de scutellaire ; une eau de fruit de kiwi (Actinidia chinensis) commercialisée par Gattefosse, un extrait de racine de Paeonia suffructicosa tel que celui commercialisé par la société Ichimaru Pharcos sous la dénomination Botanpi Liquid B® , un extrait de sucre brun (Saccharum officinarum), tel que l'extrait de melasse commercialisé par la société Taiyo Kagaku sous la dénomination Molasses Liquid, sans que cette liste soit exhaustive.

Comme agents dépigmentants préférés, on utilisera l'alpha et la béta arbutine, l'acide férulique, l'acide kojique, le résorcinol et ses dérivés, le D calcium panthéteine sulfonate, l'acide lipoique, l'acide ellagique, la vitamine B3, une eau de fruit de kiwi (Actinidia chinensis) commercialisée par Gattefosse, un extrait de racine de Paeonia suffructicosa tel que celui commercialisé par la société Ichimaru Pharcos sous la dénomination Botanpi Liquid B®.

## 5. Agents antioxydants

On peut citer notamment le tocophérol et ses esters, en particulier l'acétate de tocophérol ; l'acide férulique ; la sérine ; l'acide ellagique, la phlorétine, les polyphénols,les tanins, l'acide tannique, l'époigallocathéchines et les extraits naturels en contenant, les anthocyanes, les extraits de romarin, les extraits de feuilles d'olivier comme ceux de la société Silab, les extraits de thé vert, le resvératrol et ses dérivés, l'ergothinéine, la N acétylcystéine, un extrait d'algue brune pelvetia canaliculata comme la Pelvetiane® de Secma, l'acide chlorogénique, la biotine, les chélatants, tels que le BHT, le BHA, le N,N'-bis(3,4,5-triméthoxybenzyl) éthylenediamine et ses sels ; l'idébénone, des extraits végétaux come le Pronalen Bioprotect TM de la société Provital ; le co enzyme Q10, les bioflavonoides, les SOD, le phytantriol, les lignanes, la mélatonine, les pidolates, le gluthation, le caprylyl glycol, la phloretine, le TotarolTM ou extrait de Podocarpus totara contenant du totarol (totara-8, 11, 13-trienol ou 2-phenanthrenol, 4b, 5, 6, 7, 8, 8a, 9, 10-octahydro-4b, 8, 8-trimethyl-1(1-methylethyl)- ; un extrait de jasmin tel que celui commercialisé par SILAB sous la dénomination Helisun® ; le laurate d'hesperitine tel que le Flavagrum PEG® de la société Engelhard Lyon ; un extrait de racine de Paeonia suffructicosa tel que celui commercialisé par la société Ichimaru Pharcos sous la dénomination Botanpi Liquid B® ; un extrait de litchi tel que l'extrait de péricarpe de litchi commercialisé par la société Cognis sous la dénomination Litchiderm LS 9704®, un extrait de fruit de grenade (Punica Granatum), tel que celui commercialisé par la société Draco Natural products.

Comme autre agent anti-âge, on peut citer la DHEA et ses dérivés, l'acide boswellique, les extraits de romarin, les caroténoides (B carotène, zéaxanthine, lutéine), l'acide cystéique, les dérivés de cuivre, l'acide jasmonique

Comme agent antioxydant préféré, on utilisera notamment l'acide férulique ; la sérine ; la phlorétine, un extrait de grenade, la biotine, les chélatants, tels que le BHT, le BHA, le N,N'-bis(3,4,5-triméthoxybenzyl) éthylenediamine et ses sels , le caprylyl glycol, la phloretine, le TotarolTM, un extrait de jasmin tel que celui commercialisé par SILAB sous la dénomination Helisun® ; le laurate d'hesperitine tel que le Flavagrum PEG® de la société Engelhard Lyon ; un extrait de racine de Paeonia suffructicosa tel que celui commercialisé par la société Ichimaru Pharcos sous la dénomination Botanpi Liquid B® .

## 6. Agents dermorelaxants ou dermodécontractants

On peut citer comme exemples le gluconate de manganèse et autres sels, l'adénosine, l'alvérine citrate et ses sels, la glycine, un extrait d'Iris pallida, un hexapeptide (Argériline R de Lipotec) ou les sapogénines comme le Wild yam et les amines carbonylées décrites dans la demande EP1484052. Comme exemple de sapogénines on peut citer celles décrites dans la demande de brevet WO02/47650, en particulier le Wild yam, la diosgénine extrait notamment de Dioscorea opposita ou tout extrait refermant naturellement ou après traitement une ou plusieurs sapogénines (rhizome d'igname sauvage, feuille d'agave qui contient de l'hécogénine et la tigogénine, extrait de liliacées et plus particulièrement le Yacca ou le smilax contenant la smilagéine et la sarsapogénine, ou la racine de salsepareille) ou l' Actigen Y de la société Actives Organics ; ou le gingembre.

On peut également citer le DMAE (diméthyl MEA), les extraits de criste marine, de ciste de Montpellier, d'hélicryse, d'anis, de Para cress, un extrait d'Acmella Oleracea comme la Gatuline® expression de Gattefossé.

Comme agents dermorelaxants préférés, on citera l'adénosine, le gluconate de manganèse, le wild yam, la criste marine, la glycine et l'alvérine.

## 7. Agents anti-glycation

Par « agent anti-glycation », on entend un composé prévenant et/ou diminuant la glycation des protéines de la peau, en particulier des protéines du derme, telles que le collagène.

Comme agents anti-glycation, on peut citer notamment les extraits végétaux de la famille des Ericaceae, tels qu'un extrait de myrtille (Vaccinium angusfifollium, Vaccinium myrtillus), par exemple celui vendu sous la dénomination

« BLUEBERRY HERBASOL EXTRACT PG » par la société COSMETOCHEM, l'ergothionéine et ses dérivés, les hydroxystilbènes et leurs dérivés, tels que le resvératrol et le 3,3', 5,5'-tétrahydroxystilbène (ces agents anti-glycation sont décrits dans les demandes FR 2 802 425, FR 2 810 548, FR 2 796 278 et FR 2 802 420, respectivement), les dihydroxystilbènes et leurs dérivés, les polypeptides d'arginine et de lysine tels que celui vendu sous la dénomination « AMADORINE® » par la société SOLABIA, le chorhydrate de carcinine (commercialisé par Exsymol sous la dénomination « ALISTIN®»), un extrait d'Hélianthus annuus comme l'Antiglyskin® de Silab, les extraits de vin tel que l'extrait de vin blanc en poudre sur support maltodextrine vendu sous la dénomination « Vin blanc déshydraté 2F » par la société Givaudan, l'acide thioctique (ou acide alpha lipoïque), un mélange d'extrait de busserole et de glycogène marin comme l'Aglycal LS 8777® de Laboratoires Sériobiologiques, un extrait de thé noir comme le Kombuchka® de Sederma et leurs mélanges.

Comme agents anti-glycation préférés, on citera les extraits de myrtille (Vaccinium myrtillus) et l'extrait de thé noir.

**8. Agents stimulant la synthèse de macromolécules dermiques et/ou épidermiques et/ou empêchant leur dégradation :**

Parmi les actifs stimulant les macromolécules du derme ou empêchant leur dégradation, on peut citer ceux qui agissent :

- soit sur la synthèse du collagène, tels que les extraits de Centella asiatica, les asiaticosides et dérivés ; l'acide ascorbique ou vitamine C et ses dérivés ; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ; les hormones végétales telles que les auxines et les lignanes ; l'acide folique; et un extrait de Medicago sativa (alfafa) tel que celui commercialisé par SILBA sous la dénomination Vitanol® ; un extrait peptidique de noisette tel que celui commercialisé par la société Solabia sous la dénomination Nuteline C® ; et l'arginine.

- soit sur l'inhibition de la dégradation du collagène, en particulier des agents agissant sur l'inhibition des métalloprotéinases (MMP) telles que plus particulièrement les MMP 1, 2, 3, 9 . On peut citer : les rétinoïdes et dérivés, les extraits de medicago sativa tels que le Vitanol® de Silab, un extrait d'aphanizomenon flos-aquae (cyanophycée) commercialisée sous la dénomination Lanablue® par Atrium Biotechnologies, les oligopeptides et les lipopeptides, les lipoaminoacides, l'extrait de malt commercialisé par la société COLETICA sous la dénomination commerciale Collalift® ; les extraits de myrtille ou de romarin ; le lycopène ; les isoflavones, leurs dérivés ou les extraits végétaux en contenant, en particulier les extraits de soja (commercialisé par exemple par la société ICHIMARU PHARCOS sous la dénomination commerciale Flavostérone SB®), de trèfle rouge, de lin, de kakkon; un extrait de litchi tel que l'extrait de péricarpe de litchi commercialisé par la société Cognis sous la dénomination Litchiderm LS 9704®; la DIPALMITOYL HYDROXYPROLINE commercialisé par Seppic sous le nom SEPILIFT DPHP® : Baccharis genistelloide ou Baccharine commercialisé par SILAB, un extrait de moringa tel que l'Arganyl LS 9781® de Cognis ; l'extrait de sauge décrit dans la demande FR-A-2812544 de la famille des labiées (salvia officinalis de la société Flacksmann), l'extrait de Rhododendron, le blueberry extract, un extrait de vaccinium myrtillus tel que ceux décrits dans la demande FR-A-2814950.

- soit sur la synthèse de molécules appartenent à la famille des élastines (élastine et fibrilline), tels que : le retinol et dérivés en particulier le palmitate de rétinol ; l'extrait de Saccharomyces Cerivisiae commercialisé par la société LSN sous la dénomination commerciale Cytovitin® ; et l'extrait d'algue Macrocystis pyrifera commercialisé par la société SECMA sous la dénomination commerciale Kelpadelie® ; un extrait peptidique de noisette tel que celui commercialisé par la société Solabia sous la dénomination Nuteline C®.

- soit sur l'inhibition de la dégradation de l'élastine tels que l'extrait peptidique de graines de Pisum sativum commercialisé par la société LSN sous la dénomination commerciale Parelastyl® ; les héparinoïdes ; et les composés N-acylaminoamides décrits dans la demande WO 01/94381 tels que l'acide {2-[acétyl-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C1-C6. ; un extrait de peptides de riz tel que la Colhibin® de Pentapharm, ou un extrait de Phyllanthus emblica tel que l'Emblica® de Rona.

- soit sur la synthèse des glycosaminoglycannes, tels que le produit de fermentation du lait par lactobacillus vulgaris,

commercialisé par la société BROOKS sous la dénomination commerciale Biomin yogourth® ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin®. ; un extrait de Laminaria ochroleuca tel que la Laminaïne® de Secma ; l'essence de Mamaku de Lucas Meyer, un extrait de cresson (Odraline® de Silab).

- soit sur la synthèse de la fibronectine, tels que l'extrait de zooplancton Salina commercialisé par la société SEPORGA sous la dénomination commerciale GP4G® ;
  l'extrait de levure disponible notamment auprès de la société ALBAN MÜLLER sous la dénomination commerciale Drieline®; et le palmitoyl pentapeptide commercialisé par la société SEDERMA sous la dénomination commerciale Matrixil®.

Parmi les actifs stimulant les macromolécules épidermiques, telles que la fillagrine et les kératines, on peut citer notamment l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC; et l'extrait de zooplancton Salina commercialisé par la société SEPORGA sous la dénomination commerciale GP4G® ; Tripeptide de Cuivre de PROCYTE ; ; un extrait peptidique de Voandzeia substerranea tel que celui commercialisé par la société Laboratoires Sérobiologiques sous la dénomination commerciale Filladyn LS 9397®.

De préférence on utilisera un actif stimulant la synthèse de macromolécules dermiques et/ou épidermiques et/ou empêchant leur dégradation choisi parmi les agents stimulant la synthèse des glycosaminoglycanes, les agents inhibant la dégradation de l'élastine, les agents stimulant la synthèse de la fibronectine, les agents stimulant la synthèse de macromolécules épidermiques, et leurs mélanges.

Encore plus préférentiellement, on utilisera un actif stimulant la synthèse des glycosaminoglycanes choisi parmi un extrait d'algue brune Padina pavonica, un extrait de Saccharomyces cerevisiae, un extrait de Laminaria ochroleuca, l'essence de Mamaku, un extrait de cresson et leurs mélanges.

Comme actifs préférés stimulant la synthèse de macromolécules dermiques et/ou épidermiques et/ou empêchant leur dégradation, on peut citer :

les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ; les peptides de riz tel que le Nutripeptide® de SILAB, le méthylsilanol mannuronate tel que l'Algisium C® commercialisé par Exsymol ;; l'acide folique ; un extrait de Medicago sativa (alfafa) tel que celui commercialisé par SILBA sous la dénomination Vitanol® ; un extrait peptidique de noisette tel que celui commercialisé par la société Solabia sous la dénomination Nuteline C® ; l'arginine ; . un extrait d'aphanizomenon flos-aquae (cyanophycée) commercialisée sous la dénomination Lanablue® par Atrium Biotechnologies, l'extrait de malt commercialisé par la société COLETICA sous la dénomination commerciale Collalift® le lycopène ; un extrait de litchi ; un extrait de moringa tel que l'Arganyl LS 9781 ® de Cognis ; un extrait de vaccinium myrtillus tel que ceux décrits dans la demande FR-A-2814950 ; : le retinol et dérivés en particulier le palmitate de rétinol ; l'extrait de Saccharomyces Cerivisiae commercialisé par la société LSN sous la dénomination commerciale Cytovitin® ; un extrait peptidique de noisette tel que celui commercialisé par la société Solabia sous la dénomination Nuteline C® ; l'acide {2-[acétyt-(3-trifluorométhyl-phényl)-amino]-3-méthyl-butyrylamino} acétique, autrement nommé N-[N-acétyl, N'-(3-trifluorométhyl)phényl valyl]glycine ou N-acetyl-N-[3-(trifluoromethyl)phenyl]valyl-glycine ou acetyl trifluoromethyl phenyl valylglycine, ou un ester de celui-ci avec un alcool en C1-C6. ; un extrait de peptides de riz tel que la Colhibin® de Pentapharm, ou un extrait de Phyllanthus emblica tel que l'Emblica® de Rona ; l'extrait d'algue brune Padina pavonica commercialisé par la société ALBAN MÜLLER sous la dénomination commerciale HSP3® ; l'extrait de Saccharomyces cerevisiae disponible notamment auprès de la société SILAB sous la dénomination commerciale Firmalift® ou auprès de la société LSN sous la dénomination commerciale Cytovitin®. ; un extrait de Laminaria ochroleuca tel que la Laminaïne® de Secma ; l'essence de Mamaku de Lucas Meyer, l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC.

**9. Agents stimulant la prolifération des fibroblastes ou des kératinocytes et/ou la différenciation des kératinocytes**

Les agents stimulant la prolifération des fibroblastes utilisables dans la composition selon l'invention peuvent par exemple être choisis parmi les protéines ou polypeptides végétaux, extraits notamment du soja (par exemple un extrait de soja commercialisé par la société LSN sous la dénomination Eleseryl SH-VEG 8® ou commercialisé par la

société SILAB sous la dénomination commerciale Raffermine®) ; un extrait de protéines hydrolysées de soja tel que le RIDULISSE® de SILAB ; et les hormones végétales telles que les giberrellines et les cytokinines ; un extrait peptidique de noisette tel que celui commercialisé par la société Solabia sous la dénomination Nuteline C®.

De préférence on utilisera un agent favorisant la prolifération et/ou la différenciation des kératinocytes.

Les agents stimulant la prolifération des kératinocytes, utilisables dans la composition selon l'invention, comprennent notamment l'adénosine ; le phloroglucinol, l'extrait de feuille d'hydrangea macrophylla comme l'Amacha liquid E® d'Ichimaru Pharcos, un extrait de levure tel que le Stimoderm® de CLR ; l'extrait de Larrea divaricata tel que le Capislow® de Sederma, les mélanges d'extraits de papaye, de feuilles d'olivier et de citron tel que la Xyléine® de Vincience, l'extrait de feuille d'hydrangea macrophylla comme l'Amacha liquid E® d'Ichimaru Pharcos, le rétinol et ses esters dont le palmitate de rétinyle, le phloroglucinol, les extraits de tourteaux de noix commercialisés par Gattefosse et les extraits de solanum tuberosum tel que le Dermolectine® commercialisé par Sederma.

Parmi les agents stimulant la différenciation des kératinocytes comprennent par exemple les minéraux tels que le calcium; la criste marine, un extrait peptidique de lupin tel que celui commercialisé par la société SILAB sous la dénomination commerciale Structurine®; le beta-sitosteryl sulfate de sodium tel que celui commercialisé par la société SEPORGA sous la dénomination commerciale Phytocohésine® ; et un extrait hydrosoluble de maïs tel que celui commercialisé par la société SOLABIA sous la dénomination commerciale Phytovityl® ; un extrait peptidique de Voandzeia substerranea tel que celui commercialisé par la société Laboratoires Sérobiologiques sous la dénomination commerciale Filladyn LS 9397® ; et les lignanes tels que le sécoisolaricirésinol, le rétinol et ses esters dont le palmitate de retinyl.

Comme agents stimulant la prolifération et/ou la différenciation des kératinocytes, on peut citer encore les oestrogènes tel que l'oestradiol et homologues ; les cytokines.

Comme actifs stimulant la prolifération des fibroblastes ou des kératinocytes et/ou la différenciation des kératinocytes préférés, on citera des protéines ou polypeptides végétaux, extraits notamment du soja (par exemple un extrait de soja commercialisé par la société LSN sous la dénomination Eleseryl SH-VEG 8® ou commercialisé par la société SILAB sous la dénomination commerciale Raffermine®) ; un extrait de protéines hydrolysées de soja tel que le RIDULISSE® de SILAB ; un extrait peptidique de noisette tel que celui commercialisé par la société Solabia sous la dénomination Nuteline C® ; l'adénosine ; le phloroglucinol, un extrait de levure tel que le Stimoderm® de CLR ; un extrait peptidique de lupin tel que celui commercialisé par la société SILAB sous la dénomination commerciale Structurine®; un extrait hydrosoluble de maïs tel que celui commercialisé par la société SOLABIA sous la dénomination commerciale Phytovityl®; un extrait peptidique de Voandzeia substerranea tel que celui commercialisé par la société Laboratoires Sérobiologiques sous la dénomination commerciale Filladyn LS 9397® ; le rétinol et ses esters dont le palmitate de retinyl.

## 10. Agents favorisant la maturation de l'enveloppe cornée

On pourra utiliser dans les compositions de l'invention des agents qui interviennent sur la maturation de l'enveloppe cornée qui s'altère avec l'âge et induit une diminution de l'activité des transglutaminases. On peut citer par exemple l'urée et ses dérivés et en particulier l'Hydrovance® de National Starch et les autres actifs mentionnés dans la demande L'OREAL FR2877220 (non publiée).

## 11. Inhibiteurs de NO-synthases

L'agent ayant une action d'inhibiteur de NO synthase peut être choisi parmi les OPC (oligomères procyanidoliques) , les extraits de végétal de l'espèce Vitis vinifera notamment commercialisés par la société Euromed sous la dénomination Leucocyanidines de raisins extra, ou encore par la société Indena sous la dénomination Leucoselect®, ou enfin par la société Hansen sous la dénomination Extrait de marc de raisin ; les extraits de végétal de l'espèce Olea europaea de préférence obtenus à partir de feuilles d'olivier et notamment commercialisés par la société VINYALS sous forme d'extrait sec, ou par la société Biologia & Technologia sous la dénomination commerciale Eurol® BT ; les extraits d'un végétal de l'espèce Gingko biloba de préférence un extrait aqueux sec de ce végétal vendu par la société Beaufour sous le nom commercial Ginkgo biloba extrait standard et leurs mélanges.

## 12. Antagonistes des récepteurs périphériques des benzodiazépines (PBR)

On peut citer par exemple le 1-(2-chlorophenyl)-N-(1-methyl-propyl)-3-isoquinoline carboxamide ; les composés décrits dans les demandes WO03/030937, WO03/068753, dérivés de pyridazino[4, 5-b] indole-1-acétamide de formule générale (VII) tels que décrits dans le document WO00/44384.

## 13. Agents augmentant l'activité de la glande sébacée

On peut citer par exemple le dehydrojasmonate de méthyle, l'hécogénine, l'hédione, le le o-linoleyl-6D-glucose

et leurs mélanges.

## 14. Agents stimulant le métabolisme énergétique des cellules

L'actif stimulant le métabolisme énergétique des cellules peut par exemple être choisi parmi la biotine, un extrait de Saccharomyces cerevisiae tel que le Phosphovital® de Sederma, le mélange de sels de sodium, de manganèse, de zinc et de magnésium d'acide pyrrolidone carboxylique comme le Physiogenyl® de Solabia, un mélange de gluconate de zinc, de cuivre et de magnésium tel que le Sepitonic M3® de Seppic et leurs mélanges ; un beta-glucan issu de Saccharomyces cerevisiae tel que celui commercialisé par la société Mibelle AG Biochemistry ;

## 15. Agents tenseurs

Par « agent tenseur » utilisable selon l'invention, on entend des composés susceptibles d'avoir un effet tenseur, c'est-à-dire pouvant tendre la peau.

De manière générale on entend par agent tenseur selon l'invention tous composés solubles ou dispersibles dans l'eau à une température allant de 25°C à 50°C à la concentration de 7% en poids dans l'eau ou à la concentration maximale à laquelle ils forment un milieu d'apparence homogène et produisant à cette concentration de 7% ou à cette concentration maximale dans l'eau une rétractation de plus de 15 % dans le test décrit ci-après.

La concentration maximale à laquelle ils forment un milieu d'apparence homogène est déterminée à ± 10% près et de préférence à ± 5% près.

On entend par 'milieu d'apparence homogène' un milieu ne présentant pas d'agrégats visibles à l'oeil nu.

Pour la détermination de ladite concentration maximale, l'agent tenseur est ajouté progressivement dans l'eau sous agitation à la défloculeuse à une température allant de 25°C à 50°C, puis le mélange est maintenu sous agitation pendant une heure. On observe ensuite après 24 heures si le mélange ainsi préparé est d'apparence homogène (absence d'agrégats visibles à l'oeil nu).

L'effet tenseur peut être caractérisé par un test in vitro de rétractation.
On prépare préalablement et tel que décrit précédemment un mélange homogène de l'agent tenseur dans l'eau, à la concentration de 7% en poids ou à la concentration maximale définie précédemment.
30µl du mélange homogène est déposé sur une éprouvette rectangulaire (10x40mm , donc présentant une largeur initiale L0 de 10 mm) d'élastomère ayant un module d'élasticité de 20 MPa et une épaisseur de 100µm.
Après 3h de séchage à 22±3°C et 40±10% d'humidité relative HR, l'éprouvette d'élastomère présente une largeur rétractatée, notée L3h due à la tension exercée par l'agent tenseur déposé.

L'effet tenseur (ET) dudit agent est alors quantifié de la façon suivante :

$$\text{'ET'} = (L0 - L3h / L0) \times 100 \text{ en } \%$$

avec Lo = largeur initiale 10mm
et L3h = largeur après 3h de séchage

L'agent tenseur peut être choisi parmi :

- les protéines végétales ou animales et leurs hydrolysats ;
- les polysaccharides d'origine naturelle ;
- les silicates mixtes ;
- les particules colloïdales de charges inorganiques ;
- les polymères synthétiques ;
- et les mélanges de ceux-ci.

L'homme du métier saura choisir, dans les catégories chimiques listées ci-dessus, les matériaux répondant au test tenseur tel que décrit précédemment.

On peut citer notamment :

(a) les protéines et hydrolysats de protéines végétales, en particulier de maïs, de seigle, de froment, de sarrasin, de sésame, d'épautre, de pois, de fève, de lentille, de soja et de lupin,

(b) les polysaccharides d'origine naturelle, notamment (a) les polyholosides, par exemple (i) sous forme d'amidon issu notamment de riz, de maïs, de pomme de terre, de manioc, de pois, de froment, d'avoine, etc... ou (ii) sous

forme de carraghénanes, alginates, agars, gellanes, polymères cellulosiques et pectines, avantageusement en dispersion aqueuse de microparticules de gel, et (b) les latex constitués par la résine shellac, la gomme de sandaraque, les dammars, les élémis, les copals, les dérivés cellulosiques, et leurs mélanges,

(c) les silicates mixtes, notamment les phyllosilicates et en particulier les Laponites,

(d) les particules colloïdales de charge inorganique ayant un diamètre moyen en nombre compris entre 0,1 et 100 nm, de préférence entre 3 et 30 nm, et choisies par exemple parmi : la silice, les composites silice-alumine, l'oxyde de cérium, l'oxyde de zirconium, l'alumine, le carbonate de calcium, le sulfate de baryum, le sulfate de calcium, l'oxyde de zinc et le dioxyde de titane. Comme particules colloïdales composites silice-alumine utilisables dans les compositions selon l'invention, on peut citer par exemple celles commercialisées par la société Grace sous les noms de Ludox AM, Ludox AM-X 6021, Ludox HSA et Ludox TMA.

(e) les polymères synthétiques, tels que les latex de polyuréthanne ou les latex acrylique-silicone, en particulier ceux décrits dans la demande de brevet EP-1038519, tels qu'un polydiméthyl siloxane greffé propylthio(polyacrylate de méthyle), propylthio(polyméthacrylate de méthyle) et propylthio(polyacide méthacrylique), ou encore un polydiméthyl siloxane greffé propylthio(polyméthacrylate d'isobutyle) et propylthio(polyacide méthacrylique).

De tels polymères siliconés greffés sont notamment vendus par la Société 3M sous les dénominations commerciales VS 80, VS 70 ou LO21,

L'agent tenseur sera présent dans la composition en une quantité efficace pour obtenir l'effet biologique recherché selon l'invention.

A titre d'exemple, l'agent tenseur peut être compris dans la composition selon l'invention en une teneur allant de 0,01 à 30% en poids de matière active, de préférence de 1 % à 30% en poids de matière active, par rapport au poids total de la composition.

Par « matière active », on entend exclure le milieu dans lequel l'agent tenseur se trouve éventuellement solubilisé ou en dispersion sous sa forme commerciale, par exemple dans le cas des dispersions de particules colloïdales.

On peut également utiliser, notamment pour complémenter et/ou potentialiser l'effet d'agents tenseurs, utiliser des agents augmentant l'expression des mécanorécepteurs, tels que des agents augmentant l'expression des intégrines.

A titre d'exemple, on peut citer un extrait de graine de seigle, tel que celui commercialisé par SILAB sous la dénomination Coheliss©.

**16. Agents liporestructurants**

Par 'agents liporestrucurants', on entend selon l'invention des agents capables de stimuler la lipogénèse et favoriser la différenciation adipocytaire, permettant ainsi d'éviter ou de ralentir la fonte des graisses contenues dans les tissus de soutien de la peau, autrement nommée 'fonte de la lipo-structure de la peau'.

Par 'lipo-structure de la peau', on entend le réseau de cellules lipidiques qui forme les volumes sur lesquels la peau du visage repose et se moule.

Ces agents dont destinés à

- diminuer la perte de densité cutanée et/ou la fonte de la lipo-structure de la peau, en particulier au niveau des joues et du contour de l'oeil, et/ou
- éviter l'affaissement et/ou le creusement des volumes du visage, la perte de consistance de la peau et/ou son maintien, en particulier au niveau des joues et du contour de l'oeil, et/ou
- améliorer les volumes qui sous-tendent la peau du visage et/ou du cou, en particulier au niveau des joues, de l'ovale du visage et du contour de l'oeil, et/ou
- améliorer la densité, le rebondi et le maintien de la peau, en particulier au niveau des joues, de l'ovale du visage et du contour de l'oeil, et/ou
- refaçonner les traits du visage, en particulier l'ovale du visage.

Comme exemples d'agents liporestructurants, on peut citer notamment un extrait de thé noir, tel que l'extrait de thé noir fermenté commercialisé par Sederma sous al dénomination Kombuchka®, et un extrait d'Artemisia abrotanum, tel que celui commercialisé par SIlab sous la dénomination Pulpactyl®.

**17. Agents amincissants**

Comme agents amincissants (lipolytiques), on peut citer notamment la caféine, théophylline et ses dérivés, la

théobromine, la séricosine, l'acide asiatique, l'acéfylline, l'aminophylline, le chloroéthylthéophylline, le diprofylline, le diniprophylline, l'étamiphylline et ses dérivés, l'étofylline, la proxyphylline ; les extraits de thé, de café, de guarana, de maté, de cola (Cola Nitida) et notamment l'extrait sec de fruit de guarana (Paulina sorbilis) contenant 8 à 10 % de caféine ; les extraits de lierre grimpant (Hedera Helix), d'arnica (Arnica Montana L), de romarin (Rosmarinus officinalis N), de souci (Calendula officinalis), de sauge (Salvia officinalis L), de ginseng (Panax ginseng), de millepertuis (Byperycum Perforatum), de fragon (Ruscus aculeatus L), d'ulmaire (Filipendula ulmaria L), d'orthosiphon (Orthosiphon Stamincus Benth), de bouleau (Betula alba), de cécropia et d'arganier ; les extraits de ginkgo biloba, les extraits de prêle, les extraits d'escine, les extraits de cangzhu, les extraits de chrysanthellum indicum, les extraits de dioscorés riches en diosgénine ou la diosgénine ou hécogénine pure et leurs dérivés, les extraits de Ballote, les extraits de Guioa, de Davallia, de Terminalia, de Barringtonia, de Trema, d'Antirobia, l'extrait de petit grain de bigarrade ; un extrait de coques de fèves de cacao (theobroma cacao) tel que celui commercialisé par Solabia sous la dénomination Caobromine®.

**18. Agents favorisant la microcirculation cutanée**

L'actif agissant sur la microcirculation cutanée peut être utilisé pour éviter le ternissement du teint et/ou améliorer l'aspect du contour de l'oeil, en particulier diminuer les cernes. Il peut être choisi par exemple parmi un extrait d'écorce de pin maritime comme le Pycnogénol® de chez Biolandes, le gluconate de manganèse (Givobio GMn® de Seppic), un extrait d'Ammi visnaga comme la Visnadine d'Indena, l'extrait de lupin (Eclaline® de Silab), le couplage protéine de blé hydrolysée/acide palmitique avec acide palmitique comme l'Epaline 100 des Laboratoires carilène, l'extrait de fleur de bigarade (Remoduline® de Silab), la vitamine P et ses dérivés comme le methyl-4 esculétol mono-éthanoate de sodium vendu sous la dénomination Permethol® par la société Sephytal, les extraits de ruscus, de marron d'inde, de lierre, de ginseng et de mélilot, la caféine, le nicotinate et ses dérivés, la lysine et ses dérivés comme l'Asparlyne® de Solabia, un extrait de thé noir tel que le Kombuchka de Sederma ; les sels de rutine : un extrait d'algue de corallina officinalis tel que celui commercialisé par CODIF ; et leurs mélanges.

Comme agents préférés favorisant la microcirculation cutanée, on citera la caféine, un extrait de fleur de bigarade, un extrait de thé noir, les sels de rutine, un extrait d'algue de corallina officinalis.

**19. Agents apaisants ou anti-irritants**

On entend par agent apaisant un composé permettant de réduire la sensation de picotements, de démangeaisons ou de tiraillements de la peau.

Comme agents apaisants utilisables dans la composition selon l'invention, on peut citer : les oligomères procyannidoliques, les vitamines E, B5, B3, la caféine et ses dérivés, les triterpènes pentacycliques et les extraits de plantes les contenant, l'acide b-glycyrrhétinique et ses sels ou dérivés (le stearyl glycyrrhetate, l'acide 3-stéaroyloxy glycyrrhétique, l'acide glycyrrhétinique monoglucuronide) ainsi que les plantes en contenant (ex : Glycyrrhiza glabra), l'acide oléanolique et ses sels, l'acide ursolique et ses sels, l'acide boswellique et ses sels, l'acide bétulinique et ses sels, un extrait de Paeonia suffruticosa et/ou lactiflora, un extrait de Laminaria saccharina, les extraits de Centella asiatica, l'huile de Canola, le bisabolol, le diesterphosphorique de vitamine E et C comme le Sepivital EPC® de Seppic, les extraits de camomille, l'allantoïne, les huiles insaturées en oméga 3 telles que les huiles de rosier muscat, de cassis, d'Ecchium, de poisson, l'huile de calophilum, des extraits de plancton, la capryloyl glycine, un mélange d'extrait de fleur de nénuphar et de palmitoylproline tel que celui vendu sous la dénomination "Seppicalm VG®" par la société Seppic, un extrait de Boswellia serrata, un extrait de Centipeda cunnighami tel que celui vendu sous la dénomination "Cehami PF®" par la société TRI-K Industries, un extrait de graines de tournesol en particulier l'Hélioxine® de Silab, un extrait de graines de Linum usitatissimum comme la Sensiline® de Silab, les tocotriénols, le piperonal, un extrait d'Epilobium angustifolium tel que celui vendu sous la dénomination "Canadian Willowherb Extract" par la société FYTOKEM PRODUCTS, l'aloe vera, les phytostérols, l'eau de bleuet, l'eau de rose, un extrait de menthe, en particulier de feuilles de menthe comme le Calmiskin® de Silab, les dérivés d'anis, les bactéries filamenteuse comme Vitreoscilla filiformis tel que décrit dans le brevet EP 761 204 et commercialisé par Chimex sous la dénomination Mexoryl SBG®, un extrait de pétales de rose comme le Rose Flower Herbasol® extract de la société Cosmetochem, le beurre de karité, un mélange de fraction de cire de graine d'orge obtenue par CO2 supercritique, de beurre de karité et d'huile d'argan comme le "Stimu-tex AS®" de Pentapharm, les sels alcalino-terreux notamment le strontium, un extrait fermenté d'Alteromonas commercialisé sous la dénomination ABYSSINE® par la société Atrium Biotechnologies ; les eaux thermales du bassin de Vichy, telles que les eaux provenant des sources Célestins, Chomel, Grande-Grille, Hôpital, Lucas et Parc, et de préférence l'eau de la source Lucas ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

Comme agents apaisants préférés selon l'invention, on utilisera :

l'acide b-glycyrrhétinique et ses sels ou dérivés (le stearyl glycyrrhetate, l'acide 3-stéaroyloxy glycyrrhétique, l'acide

glycyrrhétinique monoglucuronide) ainsi que les plantes en contenant (ex : Glycyrrhiza glabra) ; l'acide ursolique et ses sels ; les extraits de Centella asiatica, l'huile de Canola, le bisabolol ; les extraits de camomille, l'allantoïne ; un mélange d'extrait de fleur de nénuphar et de palmitoylproline tel que celui vendu sous la dénomination "Seppicalm VG®" par la société Seppic ; l'aloe vera, l'eau de rose, un extrait de menthe, en particulier de feuilles de menthe comme le Calmiskin® de Silab, les bactéries filamenteuse comme Vitreoscilla filiformis tel que décrit dans le brevet EP 761 204 et commercialisé par Chimex sous la dénomination Mexoryl SBG®, un extrait de pétales de rose comme le Rose Flower Herbasol® extract de la société Cosmetochem, le beurre de karité, un extrait fermenté d'Alteromonas commercialisé sous la dénomination ABYSSINE® par la société Atrium Biotechnologies ; les eaux thermales du bassin de Vichy, telles que les eaux provenant des sources Célestins, Chomel, Grande-Grille, Hôpital, Lucas et Parc, et de préférence l'eau de la source Lucas ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

**20. Agents sébo-réaulateurs ou anti-séborrhéiques**

Par agents "séborégulateurs ou anti-séborrhéiques ", on entend notamment des agents capables de réguler l'activité des glandes sébacées.

On peut citer notamment :

- l'acide rétinoïque, le peroxyde de benzoyle, le soufre, la vitamine B6 (ou pyridoxine ), le chlorure de sélénium, la criste marine ;
- les mélanges d'extrait de canelle, de thé et d'octanoylglycine tel que le Sepicontrol A5 TEA® de chez Seppic ;
- le mélange de canelle, de sarcosine et d'octanoylglycine, commercialisé notamment par la société SEPPIC sous la dénomination commerciale Sepicontrol A5® ;
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les dérivés de cuivre et en particulier le pidolate de cuivre tel que Cuivridone® de Solabia ;
- des extraits de végétaux des espèces Arnica montana, Cinchona succirubra, Eugenia caryophyllata, Humulus lupulus, Hypericum perforatum, Mentha piperita, Rosmarinus officinalis, Salvia oficinalis et Thymus vulgaris, tous commercialisés par exemple par la société MARUZEN ;
- les extraits de reine des prés (spiraea ulamaria) tel que celui vendu sous la dénomination Sébonormine® par la société Silab ;
- les extraits d'algue laminaria saccharina tel que celui vendu sous la dénomination Phlorogine® par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- les extraits de graines de lin tel que celui vendu sous la dénomination Linumine® par la société Lucas Meyer ;
- les extraits de Phellodendron tels que ceux vendu sous la dénomination Phellodendron extract BG par la société Maruzen ou Oubaku liquid B par la société Ichimaru Pharcos ;
- les mélanges d'huile d'argan, d'extrait de serenoa serrulata (saw palmetto) et d'extrait de graines de sésame tel que celui vendu sous la dénomination Regu SEB® par la société Pentapharm ;
- les mélanges d'extraits d'epilobe, de terminalia chebula, de capucine et de zinc biodisponible (microalgues) tel que celui vendu sous la denomination Seborilys® par la société green tech ;
- les extraits de Pygeum afrianum tel que celui vendu sous la dénomination Pygeum afrianum sterolic lipid extract par la société Euromed ;
- les extraits de serenoa serrulata tels que ceux vendus sous les dénomination Viapure Sabal par la société Actives International, ou ceux vendus par la société Euromed ;
- les mélanges d'extraits de plantain, de berberis aquifolium et de salicylate de sodium tels que celui vendu sous la dénomination Seboclear® par la société Rahn ;
- l'extrait de clou de girofle tel que celui vendu sous la dénomination Clove extract Powder par la société Maruzen ;
- l'huile d'argan telle que celle vendue sous la dénomination Lipofructyl® par les Laboratoires Sérobiologiques ;
- les filtrats de protéine lactique tels que celui vendu sous la dénomination Normaseb® par la société Sederma ;
- les extraits d'algue laminaria, tel que celui vendu sous la dénomination Laminarghane® par la société Biotechmarine ;
- les oligosaccharides d'algue laminaria digitata tel que celui vendu sous la dénomination Phycosaccharide AC par la société Codif ;
- les extraits de sucre de canne tel que celui commercialisé sous la dénomination Policosanol® par la société Sabinsa ;
- l'huile de schiste sulfonée, telle que celle vendue sous la dénomination Ichtyol Pale® par la société Ichthyol ;

- les extraits d'ulmaire (spiraea ulmaria) tels que celui vendu sous la dénomination Cytobiol® Ulmaire par la société Libiol ;
- l'acide sébacique, notamment vendu sous la forme d'un gel de polyacrylate de sodium sous la dénomination Sebosoft® par la société Sederma ;
- les glucomannanes extraits de tubercule de konjac et modifié par des chaînes alkylsulfonates tel que celui vendu sous la dénomination Biopol Beta par la société Arch Chemical ;
- les extraits de Sophora angustifolia, tels que ceux vendus sous la dénomination Sophora powder ou Sophora extract par la société Bioland ;
- les extraits de cinchona succirubra bark tel que celui vendu sous la dénomination le Red bark HS par la société Alban Muller ;
- les extraits de quillaja saponaria tel que celui vendu sous la dénomination Panama wood HS par la société Alban Muller ;
- la glycine greffée sur chaîne undécylénique, telle que celle vendue sous la dénomination Lipacide UG OR par la société Seppic ;
- le mélange d'acide oléanolique et d'acide nordihydroguaïarétique, tel que celui vendus sous la forme d'un gel sous la dénomination AC.Net par la société Sederma ;
- l'acide phthalimidoperoxyhexanoïque ;
- le citrate de trialkyle(C12-C13) vendu sous la dénomination COSMACOL® ECI par la société Sasol ; le citrate de trialkyle(C14-C15) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ; et
- leurs mélanges.

Comme actif anti-séborrhéique préférés, on peut citer :

- le peroxyde de benzoyle, la vitamine B6 (ou pyridoxine ),
- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ;
- les extraits de reine des prés (spiraea ulamaria) tel que celui vendu sous la dénomination Sébonormine® par la société Silab ;
- les extraits d'algue laminaria saccharina tel que celui vendu sous la dénomination Phlorogine® par la société Biotechmarine ;
- les mélanges d'extraits de racines de pimprenelle (sanguisorba officinalis/poterium officinale), de rhizomes de gingembre (zingiber officinalis) et d'écorce de cannelier (cinnamomum cassia) tel que celui vendu sous la dénomination Sebustop® par la société Solabia ;
- l'extrait de clou de girofle tel que celui vendu sous la dénomination Clove extract Powder par la société Maruzen ;
- les filtrats de protéine lactique tels que celui vendu sous la dénomination Normaseb® par la société Sederma ;
- les extraits d'ulmaire (spiraea ulmaria) tels que celui vendu sous la dénomination Cytobiol® Ulmaire par la société Libiol ;
- l'acide sébacique, notamment vendu sous la forme d'un gel de polyacrylate de sodium sous la dénomination Sebosoft® par la société Sederma ;
- la glycine greffée sur chaîne undécylénique, telle que celle vendue sous la dénomination Lipacide UG OR par la société Seppic ;
- le citrate de trialkyle(C12-C13) vendu sous la dénomination COSMACOL® ECI par la société Sasol ; le citrate de trialkyle(C14-C15) vendu sous la dénomination COSMACOL® ECL par la société Sasol ;
- l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ;
- et leurs mélanges.

Préférentiellement, l'actif anti-séborrhéique est choisi parmi :

- les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ; et de préférence le pyrrolidone carboxylate de zinc (ou pidolate de zinc) ou le salicylate de zinc ;
- l'extrait de clou de girofle tel que celui vendu sous la dénomination Clove extract Powder par la société Maruzen ;
- la glycine greffée sur chaîne undécylénique, telle que celle vendue sous la dénomination Lipacide UG OR par la société Seppic ;
- le citrate de trialkyle(C12-C13) vendu sous la dénomination COSMACOL® ECI par la société Sasol ; le citrate de

trialkyle(C14-C15) vendu sous la dénomination COSMACOL® ECL par la société Sasol ;
- et leurs mélanges.

L'actif anti-séborrhéique est par exemple présent dans une teneur allant de 0,1 à 10 % en poids, de préférence de 0,1 à 5 % en poids, et préférentiellement de 0,5 % à 3 % en poids, par rapport au poids total de la composition.

**21. Agents astringents**

Par "agents astringents", on entend selon l'invention des agents permettant de lutter contre la dilatation des follicules sébacés.

Comme agents astringents utilisables dans la composition selon l'invention, on peut citer des extraits de pulpe de champignon (polyporus officinalis) comme le "Laricyl LS8865® " de Cognis, des extraits de Terminalia catappa et sambucus nigra comme le Phytofirm LS9120® de Cognis, des extraits de noix de galle comme le Tanlex VE® de Ichimaru Pharcos, l'hydroxychlorure d'aluminium, les extraits de centella (ex Plantactiv centella de Cognis), le chlorure de dicétyl diméthyl ammonium comme le Varisoft 432 CG® de Degussa, les extraits de marron d'inde, les extraits de mauve, les extraits d'Hammamelis, des extraits d'amandes douces, de racines de guimauve et de graines de lin comme l'Almondermin LS 3380® de Cognis, les extraits de bardane, les extraits d'ortie, les extraits de bouleau, les extraits de prêle, les extraits de camomille comme ceux vendus sous la dénomination Extrapone 9 special® par la société Symrise, les extraits de scutellaria, les extrais d'Ulmaire (par exemple le Cytobiol Ulmaire de Libiol), un mélange d'extraits de gingembre blanc, de prêle, d'ortie, de romarin, de yucca comme l'Herb extract B1348® de Bell flavors & fragrances, les extraits d'acacia, d'orme, de saule blanc, de canelle, de bouleau, de reine des prés, les sapogénines de panama, le phenolsulfonate de zinc de Interchemical, des extraits de gentiane, de concombre, de noyer, le mélange d'extraits de Ratanhia, de pamplemousse, de grindelia et de galle de chêne comme l'Epilami® de Alban Muller.

Comme agents astringents préférés selon l'invention, on utilisera les extraits de scutellaria, les extraits d'ulmaire, les extraits de reine des près, les extraits de gentiane, les extraits de bardane et leurs mélanges.

**22. Agents cicatrisants**

Comme exemples d'agents cicatrisants, on peut citer notamment :
l'allantoine, l'urée, certains acides aminés comme l'hydroxyproline, l'arginine, la sérine, et aussi des extraits de lys blanc (comme le Phytélène Lys 37EG 16295 de Indena), un extrait de levures comme le cicatrisant LS LO/7225B des Laboratoires Sériobiologiques), l'huile de tamanu, l'extrait de saccharomyces cerevisiae comme le Biodynes® TRF® de Arch Chemical, les extraits d'avoine, le chitosane et dérivés comme le glutamate de chitosane, les extraits de carotte, l'extrait d'artemia comme le GP4G® de Vincience, l'acexamate de sodium, des extraits de lavandin, des extraits de propolis, l'acide ximeninique et ses sels, l'huile de rosa rugosa, des extraits de souci comme le Souci Ami® Liposolible d'Alban Muller, des extraits de prêle, les extraits d'écorce de citron comme l'Herbasol® citron de Cosmetochem, des extraits d'helichryse, des extraits de millefeuilles, et l'acide folique.

Comme agents cicatrisants préférés selon l'invention, on utilisera l'arginine, la sérine, l'acide folique, l'huile de tamanu, l'acexamate de sodium, des extraits de prêle, des extraits d'helichryse, et leurs mélanges.

**23. Agents anti-inflammatoires**

Comme agents anti-inflammatoires particuliers utilisables selon l'invention, on peut citer la cortisone, l'hydrocortisone, l'indométhacine, la bétaméthasone, l'acide azéalique, l'acétominophène, le diclofénac, le propionate de clobetasol, l'acide folique ; un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

Comme agent anti-inflammatoire préféré, on citera l'acide azélaique, l'acide folique, un extrait d'écorce d'Eperua falcata tel que celui commercialisé par la société COGNIS sous al dénomination Eperuline® ; un extrait de racine de Paeonia suffructicosa tel que celui commercialisé par la société Ichimaru Pharcos sous la dénomination Botanpi Liquid B® ; et leurs mélanges.

**24. Agents anti-acné**

Dans un aspect avantageux de l'invention, la composition peut comporter en outre au moins un actif anti-acné.

Par "actif anti-acné", on entend notamment tout actif ayant des effets sur la flore spécifique des peaux grasses tels que par exemple le Propionibacterium acnes (P acnes).

Ces effets peuvent être bactéricides.

A titre d'actifs antibactéricide, on peut citer notamment :

- les actifs et conservateurs à activité anti-microbienne cités dans la demande DE10324567, incorporée dans la présente invention par référence,
- l'acide asiatique,
- le sel de monoéthanolamine du 1-hydroxy-4-methyl 6-trimethylpentyl 2-pyridone (nom INCI: piroctone olamine), notamment vendu sous la dénomination Octopirox® par la société Clariant ;
- l'acide citronellique, l'acide périllique (ou acide 4-isopropenylcyclohex-1-ènecarboxylique ),
- le 2-éthyl hexyle éther de glycérol (nom INCI : ethylhexylglycerine), par exemple vendu sous la dénomination Sensiva SC 50® par la société Shulke & Mayr,
- le caprylate/caprate de glyceryle, par exemple vendu sous la dénomination Capmul MCM® par la société ABITEC ;
- le phosphosilicate de calcium et de sodium, notamment vendu sous les dénominations Bioactive glasspowder® et Actysse Premier BG® par la société Schott Glass ;
- les particules à base d'argent, par exemple celle vendues sous la dénomination Métashine ME 2025 PS® par la société Nippon Sheet Glass ;
- l'extrait de cône de houblon (Humulus Lupulus) obtenu par extraction CO2 supercritique tel que celui vendu sous la dénomination HOP CO2-TO extract® par la société Flavex Naturextrakte,
- l'extrait de Millepertius obtenu par extraction CO2 supercritique tel que celui vendu sous la dénomination ST John's Wort CO2-TO extract® par la société Flavex Naturextrakte,
- le mélange d'extraits de racines de scutellaria baicalensis, de paeonia suffruticosa et de glycyrrhiza glabra, tel que celui vendu sous la dénomination BMB - CF® par la société Naturogin,
- l'extrait d'arganier comme l'Argapure LS9710® de chez COGNIS ;
- les extraits de feuilles de busserole comme celui vendu sous la dénomination "Melfade-J" par la société Pentapharm ;
- l'acide 10-hydroxy-2 décanoique tel que l'Acnacidol P® de chez Vincience, l'ursolate de sodium, l'acide azélaique, le di-iodo-méthyl p-tolylsulfone tel que l'Amical Flowable® de chez Angus, la poudre de malachite, l'oxyde de zinc tel que Zincare® de chez Elementis GMBH, l'acide octadécènedioïque tel que l'Arlatone dioic DCA® de chez Uniqema ; l'acide ellagique ; le 2,4,4'-trichloro-2'-hydroxy diphényl éther (ou triclosan), la 1-(3',4'-dichlorophényl)-3-(4'-chlorophényl)urée (ou triclocarban), le 3,4,4'-trichlorocarbanilide, le 3',4',5'-trichlorosalicylanilide, le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopirox, le ciclopiroxolamine, l'acide undécylenique et ses sels, le peroxyde de benzoyle, l'acide 3-hydroxy benzoïque, l'acide 4-hydroxy benzoïque, l'acide phytique, la N-acétyl-L-cystéine, l'acide lipoïque, l'acide azélaïque et ses sels, l'acide arachidonique, le résorcinol, le 3,4,4'-trichlorocarbanalide, l'octoxyglycérine ou octoglycérine, l'octanoylglycine tel que Lipacid C8G® de Seppic, le caprylyl glycol, l'acide 10-hydroxy-2-décanoïque, le dichlorophenyl imidazol dioxolan et ses dérivés décrits dans la demande de brevet WO9318743, l'iodopropynyl butylcarbamate, le 3,7,11-triméthyldodéca-2,5,10-triénol ou farnesol, les phytosphingosines ; les sels d'ammonium quaternaires comme les sels de cetyltrimethylammonium, les sels de cétylpyridinium, et
- leurs mélanges.

On peut également citer certains tensioactifs ayant un effet antimicrobien comme le cocoampho acétate de sodium ou diacetate disodium tel que le Miranol C2M CONC NP, les bétaines comme la cocoyl bétaine Genagen KB de Clariant, le lauryl ether sulfate de sodium comme l'Emal 270 D de Kao, le décyl glucoside comme le Plantacare 2000 UP, les malate de dialcools C12-13 ramifiés comme le Cosmacol EMI, les monoesters de propylène glycol comme monolaurate, monocaprylate, monocaprate de propylène glycol, le lauryl dimethylamine betaine comme le Empigen BB/LS ainsi que les polyammonium quaternaires comme le Quaternium-24 ou Bardac 2050 de Lonza et ceux décrits dans le brevet FR 0 108 283 et leurs mélanges.

Comme agents antimicrobiens préférés, on utilisera dans les compositions de l'invention un agent choisi parmi l'octoglycérine ou octoxyglycérine, l'acide 10-hydroxy-2-décanoïque, et leurs mélanges.

D'autres actifs anti-acné additionnels peuvent être ajoutés aux actifs anti-acnés précités.

On peut notamment citer les actifs présentant des effets anti-adhésion bactérienne ou bien agissant sur le biofilm des bactéries pour éviter leur multiplication.

Comme agents prévenant et/ou réduisant l'adhésion des micro-organismes, on peut citer notamment : le phytanetriol et ses dérivés tels que décrits dans la demande de brevet EP 1 529 523, les huiles végétales telles de l'huile de germe de blé, l'huile de calendula, l'huile de ricin, l'huile d'olive, l'huile d'avocat, l'huile d'amande douce, l'huile d'arachide, l'huile de jojoba, l'huile de sésame, l'huile d'amande d'abricot, l'huile de tournesol, l'huile de macadamia, décrites dans le brevet EP 1 133979, ou encore certains tensioactifs tels que le cocoamphodiacétate disodique, le cocoate de glycéryle oxyéthyléné (7 OE), l'hexadécénylsuccinate 18, le palmitate d'octoxyglycéryle, le béhénate d'octoxyglycéryle, l'adipate de dioctyle, le PPG-15 stéaryl éther, le tartrate de dialcools C12-C13 ramifiés décrits dans le brevet EP 1 129 694 et leurs mélanges. En particulier vis-à-vis de la propagation du P acnes, ou en tant qu'actifs agissant sur le biofilm des bactéries pour éviter leur prolifération, on peut citer le pentylène glycol, le nylon-66 (fibres de polyamides 66), l'huile de son de riz , l'alcool polyvinylique tel que Celvol 540 PV alcohol® de chez Celanese Chemical, l'huile de colza telle que Akorex L® de chez Karlshamns, et les dérivés de fructose et leurs mélanges.

L'actif agissant contre l'acné peut être présent dans une teneur allant de 0,01 à 10 % en poids, de préférence de 0,05 à 5 % en poids par rapport au poids total de la composition.

En fonction de la nature et/ou de la solubilité des actifs précités, l'homme du métier saura choisir le mode de réalisation le plus adapté selon l'invention.

Les actifs cosmétiques et/ou dermatologiques seront présents dans l'une des compositions selon l'invention en une teneur allant de 0,001 % à 20% en poids par rapport au poids total de la composition, de préférence de 0,01% à 10%, encore plus préférentiellement de 0,5 à 5% et de préférence encore de 0,1 à 1% en poids par rapport au poids total de la composition.

Pour des applications 'peeling', les teneurs en actifs cosmétiques et/ou dermatologiquies pourront aller de 1 à 50% en poids par rapport au poids total de la composition, de préférence de 1 à 30% en poids par rapport au poids total de la composition.

Les peelings sont un moyen bien connu pour améliorer l'aspect et/ou la texture de la peau et/ou du cuir chevelu, notamment améliorer l'éclat et l'homogénéité du teint et/ou diminuer les irrégularités visibles et/ou tactiles de la peau, et en particulier pour améliorer l'aspect de surface de la peau, pour atténuer les lentigo actiniques, les marques d'acné ou de varicelle, ainsi que pour prévenir, atténuer ou lutter contres les signes du vieillissement cutané, et notamment pour lisser les irrégularités de la texture de la peau, telles les rides et les ridules.

Ils ont pour effet d'enlever une partie superficielle de la peau à traiter (épiderme et éventuellement couche superficielle du derme), par des méthodes chimiques.

<u>AUTRES INGREDIENTS ADDITIONNELS</u>

Pour complémenter et/ou optimiser les effets conférés par les actifs cosmétiques et/ou dermatologiques cités ci-dessus sur les matières kératiniques, il peut être avantageux d'intégrer dans les compositions de l'invention d'autres ingrédients additionnels.

En particulier, ces ingrédients addditionnels pourront conférer un effet immédiat visuel qui sera relayé par l'effet biologique des actifs cités ci-dessus.

Ils pourront également, via une action mécanique (ex : charges abrasvies), amplifier l'effet des actifs biologiques cités ci-dessus.

Ainsi la composition selon l'invention pourra comprendre en outre au moins un agent choisi parmi des agents matifiants, des charges à effet flouteur, des agents fluorescents, des agents favorisant la coloration naturellement rosée de la peau, des charges abrasives ou agents exfoliants, et leurs mélanges.

**<u>Agents matifiants</u>**

Par "agent matifiant", on entend des agents destinés à rendre la peau visiblement plus mate, moins brillante.

L'effet matifiant de l'agent et/ou de la composition le contenant peut notamment être évalué à l'aide d'un gonioréflectomètre, en mesurant le rapport R entre la réflexion spéculaire et la réflexion diffuse. Une valeur de R inférieure ou égale à 2 traduit généralement un effet matifiant.

L'agent matifiant pourra notamment être choisi parmi un amidon de riz ou un amidon de maïs, la kaolinite, le talc, un extrait de graines de potiron, des microbilles de cellulose, des fibres végétales, des fibres synthétiques, en particulier de polyamides, des microsphères de copolymères acryliques expansées, des poudres de polyamides, les poudres de silice, les poudres de polytétrafluoroéthylène, les poudres de résine de silicone, les poudres de polymères acryliques, les poudres de cire, les poudres de polyéthylène, les poudres d'organopolysiloxane réticulé élastomère enrobées de résine de silicone, les poudres composites de talc/dioxyde de titane/alumine/silice, les poudres de silicates mixtes amorphes, les particules de silicate et notamment de silicate mixte, et leurs mélanges.

Comme exemples d'agents matifiants, on peut citer notamment :

- l'amidon de riz ou de maïs, en particulier un aluminium starch octenyl succinate commercialisé sous la dénomination Dry Flo® par la société National Starch,
- la kaolinite ;
- les silices ;
- le talc ;
- un extrait de graines de potiron tel que commercialisé sous la dénomination Curbilene® par la société Indena ;

- des microbilles de cellulose telles que décrites dans la demande de brevet EP 1 562 562 ;
- des fibres, telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de Téflon®, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742 ;
- des microsphères de copolymères acryliques expansées telles que celles commercialisées par la société EXPANCEL sous les dénominations EXPANCEL 551®,
- des charges à effet optique telles que décrites dans la demande de brevet FR 2 869 796, en particulier :
- les poudres de polyamides (Nylon®), comme par exemple les particules de Nylon 12 du type Orgasol d'Arkema de taille moyenne 10 microns et d'indice de réfraction 1,54,
- les poudres de silice, comme par exemple les Silica beads SB150 de Miyoshi de taille moyenne 5 microns et d'indice de réfraction 1,45,
- les poudres de polytétrafluoroéthylène, comme les PTFE ceridust 9205F de Clariant de taille moyenne 8 microns et d'indice de réfraction 1,36,
- les poudres de résine de silicone comme les Silicon resin Tospearl 145A de GE Silicone de taille moyenne 4,5 microns et d'indice de réfraction 1,41,
- les poudres de copolymères acryliques, notamment de poly(méth)acrylate de méthyle, comme les particules PMMA Jurymer MBI de Nihon Junyoki de taille moyenne 8 microns et d'indice de réfraction 1,49, ou les particules Micropearl M100® et F 80 ED® de la société Matsumoto Yushi-Seiyaku,
- les poudres de cire comme les particules Paraffin wax microease 114S de micropowders de taille moyenne 7 microns et d'indice de réfraction 1,54,
- les poudres de polyéthylène, notamment comprenant au moins un copolymère éthylène/acide acrylique, et en particulier constituées de copolymères éthylène/acide acrylique comme les particules Flobeads EA 209 de Sumitomo (de taille moyenne 10 microns et d'indice de réfraction 1,48),
- les poudres d'organopolysiloxane réticulé élastomère enrobées de résine de silicone, notamment de résine silsesquioxane, comme décrit par exemple dans le brevet US 5 538 793. De telles poudres d'élastomère sont vendues sous les dénominations "KSP-100", "KSP-101", "KSP-102", "KSP-103", "KSP-104", "KSP-105" par la société SHIN ETSU, et
- les poudres composites de talc/dioxyde de titane/alumine/silice comme celles vendues sous la dénomination Coverleaf® AR-80 par la société Catalyst & chemicals,
- leurs mélanges,
- des composés absorbant et/ou adsorbant le sébum tels que décrits dans la demande de brevet FR 2 869 796. On peut citer notamment :
- les poudres de silice, comme par exemple les microsphères de silice poreuses vendues sous la dénomination "SILICA BEADS SB-700" commercialisées par la société MYOSHI, les "SUNSPHERE® H51", "SUNSPHERE® H33", "SUNSPHERE® H53" commercialisées par la société ASAHI GLASS ; les microsphères de silice amorphe enrobées de polydiméthylsiloxane vendues sous la dénomination "SA SUNSPHERE® H-33" et "SA SUNSPHERE® H-53" commercialisées par la société ASAHI GLASS ;
- les poudres de silicates mixtes amorphes, notamment d'aluminium et de magnésium, comme par exemple celle commercialisée sous la dénomination "NEUSILIN UFL2" par la société Sumitomo.
- les poudres de polyamides (nylon®), comme par exemple "l'ORGASOL® 4000" commercialisé par la société Arkema, et
- les poudres de polymères acryliques, notamment de polyméthacrylate de méthyle, comme par exemple le "COVABEAD® LH85" commercialisé par la société WACKHERR ; de polyméthacrylate de méthyle/diméthacrylate d'éthylène glycol, comme par exemple le "DOW CORNING 5640 MICROSPONGE® SKIN OIL ADSORBER" commercialisé par la société DOW CORNING, ou le "GANZPEARL® GMP-0820" commercialisé par la société GANZ CHEMICAL ; de polyméthacrylate d'allyle/diméthacrylate d'éthylène glycol, comme par exemple le "POLY-PORE® L200" ou le "POLY-PORE® E200" commercialisés par la société AMCOL ; de copolymère diméthacrylate d'éthylène glycol/méthacrylate de lauryle, comme par exemple le "POLYTRAP® 6603" commercialisé de la société DOW CORNING ;
- les particules de silicate, telle que la silicate d'alumine ;
- les particules de silicates mixtes, telles que :

- les particules de silicate d'aluminium et de magnésium, telles que la saponite ou silicate de magnésium et d'aluminium hydraté avec un sulfate de sodium commercialisée sous la dénomination commerciale Sumecton® par la société Kunimine ;
- le complexe silicate de magnésium, hydroxyéthylcellulose, huile de cumin noir, huile de courge et phospholipides

ou Matipure® de Lucas Meyer, et
- leurs mélanges.

Comme agents matifiants préférés, on pourra utiliser selon l'invention un extrait de graines de potiron, un amidon de riz ou de mais, la kaolinite, des silices, le talc, les poudres de polyamides, les poudres de polyéthylènes, les poudres de copolymères acryliques, les microsphères de copolymères acryliques expansées, les microbilles de résines de silicones, les particules de silicate mixte et leurs mélanges.

**Charges à effet flouteur**

Ces charges peuvent être tout matériau susceptible de modifier les rides par ses propriétés physiques intrinsèques et de les masquer. Ces charges peuvent notamment modifier les rides par un effet tenseur, un effet de camouflage, ou un effet de floutage.
En tant que charge, on peut donner à titre d'exemples les composés suivants :

- les microparticules poreuses de silice comme par exemple les Silica Beads® SB 150 et SB 700 de Myochi de taille moyenne de 5μm et les SUNSPHERES® série H d'Asahi Glass comme les H33, H51 de taille respectivement de 3,5 et 5 μm.
- les particules hémisphériques creuses de résines de silicones comme les NLK 500®, NLK 506® et NLK 510® de Takemoto Oil and Fat, notamment décrites dans EP-A-1579849,
- les poudres de résine de silicone comme par exemple les SILICON Resin Tospearl® 145 A DE GE silicone de taille moyenne de 4,5μm.
- les poudres de copolymères acryliques, notamment de poly(meth)acrylate de méthyle comme par exemple les particules PMMA Jurimer MBI® de Nihon Junyoki de taille moyenne de 8μm, les sphères creuses de PMMA vendues sous la dénomination COVABEAD® LH 85 par la société Wackherr et les microsphères de vinylidène/acrylonitrile/méthacrylates de méthylène expansées vendues sous la dénomination Expancel®.
- les poudres de cires comme les particules Paraffin wax microloase® 114S de Micropowders de taille moyenne de 7μm.
- les poudres de polyéthylènes notamment comprenant au moins un copolymère éthylène/acide acrylique comme par exemple les FLOBEADS® EA 209 E de Sumimoto de taille moyenne de 10μm.
- les poudres d'organopolysiloxanes élastomériques réticulées enrobées de résine de silicone notamment de silsesquioxane sous la dénomination KSP 100®, KSP 101®, KSP 102®, KSP 103®, KSP 104® et KSP 105® par la société Shin Etsu.
- les poudres composites de talc/dioxyde ou de titane/alumine/silice comme par exemple les Cverleaf AR 80® de la société Catalyst & Chemical.
- le talc, le mica, le kaolin, la lauryl glycine, les poudres d'amidon réticulés par l'anhydride octéanyl succinate, le nitrure de bore, les poudres de polytétrafluoroéthylène, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques.
- les fibres hydrophiles ou hydrophobes synthétiques ou naturelles, minérales ou organiques telles que des fibres de soie, de coton, de laine, de lin, de cellulose extraites notamment du bois, des légumes ou des algues, de polyamide (Nylon®), de cellulose modifiée, de poly-p-phénylène téréphtamide, en acrylique, de polyoléfine, de verre, de silice, d'aramide, de carbone, de polytétrafluoroéthylène (Téflon®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères, les fibres synthétiques résorbables, et leurs mélanges décrites dans la demande de brevet EP 1 151 742.
- les silicones réticulés élastomères sphériques comme comme les Trefil E-505C® ou E-506 C® de chez Dow Corning.
- les charges abrasives qui par effet mécanique apportent un lissage du microrelief cutané, telles que la silice abrasive comme par exemple Abrasif SP® de Semanez ou des poudres de noix ou de coques (abricot, noix par exemple de Cosmétochem).

Les charges ayant un effet sur les signes du vieillissement sont notamment choisies parmi des microparticules poreuse de silice, des particules hémisphériques creuses de silicones, des poudres de résine de silicone, des poudres de copolymères acryliques, des poudres de polyéthylènes, des poudres d'organopolysiloxanes élastomériques réticulées enrobées de résine de silicone, des poudres composites de talc/dioxyde de titane/alumine/silice, le carbonate de calcium précipité, le carbonate de l'hydrocarbonate de magnésium, le sulfate de baryum, l'hydroxyapatite, le silicate de calcium, le dioxyde de cérium et les microcapsules de verre ou de céramiques, les fibres de soie, de coton, et leurs mélanges.
La charge peut être une charge « soft focus ».

Par charge « soft-focus », on entend une charge qui en plus donne de la transparence au teint et un effet flou. De préférence, les charges « soft-focus » ont une taille moyenne des particules inférieure ou égale à 15 microns. Ces particules peuvent être de toutes formes et en particulier être sphériques ou non sphériques. De préférence encore, ces charges sont non sphériques.

Les charges « soft-focus » peuvent être choisies parmi les poudres de silice et silicates, notamment d'alumine, les poudres de type polyméthyl méthacrylate (PMMA), le talc, les composites silice/$TiO_2$ ou silice/oxyde de zinc, les poudres de polyéthylène, les poudres d'amidon, les poudres de polyamides, les poudres de copolymères styrène/acrylique, les élastomères de silicone, et leurs mélanges.

En particulier, on peut citer le talc de taille moyenne en nombre inférieure ou égale à 3 microns, par exemple du talc de taille moyenne en nombre de 1,8 micron et notamment celui vendu sous la dénomination commerciale Talc P3® par la société Nippon Talc, la poudre de Nylon® 12, notamment celle vendue sous le dénomination Orgasol 2002 Extra D Nat Cos® par la société Atochem, les particules de silice traitées en surface par une cire minérale 1 à 2 % (nom INCI : hydrated silica (and) paraffin) telles que celles commercialisées par la société Degussa, les microsphères de silice amorphe, telles que celles vendues sous la dénomination Sunsphère par exemple de référence H-53® par la société Asahi Glass, et les micro-billes de silice telles que celles vendues sous la dénomination SB-700® ou SB-150® par la société Miyoshi, cette liste n'étant pas limitative.

La concentration de ces charges ayant un effet sur les signes du vieillissement dans les compositions selon l'invention peut être comprise entre 0,1 et 40 %, voire entre 0,1 et 20 % en poids par rapport au poids total de la composition.

## Agents fluorescents

On entend par agent fluorescent une substance qui, sous l'effet de rayons ultraviolet et/ou de la lumière visible, ré-émet dans le visible la portion de lumière qu'elle a absorbé sous la même couleur que celle qu'elle reflète naturellement. La couleur réfléchie naturellement est ainsi renforcée par la couleur ré émise et apparaît extrêmement brillante.

On peut citer par exemple les résines colorées de polyamide et/ou de formaldéhyde/benzoguanamine et/ou de melamine/formaldéhyde/sulfonamide, parmi les co-condensats aminotriazine/formaldéhyde/sulfonamide colorés et/ou parmi les paillettes polyester métallisées et/ou leurs mélanges. Ces pigments fluorescents peuvent aussi se présenter sous la forme de dispersions aqueuses de pigments fluorescents.

On peut encore citer le co-condensat aminotriazine/formaldehyde/sulfonamide fluorescent coloré en rose de taille moyenne des particules 3-4 microns vendu sous la dénomination commerciale « Fiesta Astral Pink FEX-1 » et le co-condensat aminotriazine/formaldehyde/sulfonamide fluorescent coloré en bleu de taille moyenne des particules 3-4,5 microns vendu sous la dénomination commerciale « Fiesta Comet Blue FTX-60 » par la société Swada ou encore la résine benzoguanamine/formaldéhyde recouverte de résine formaldéhyde/urée et colorée en jaune vendue sous la dénomination commerciale « FB-205 Yellow » et la résine benzoguanamine/formaldéhyde recouverte de résine formaldéhyde/urée et colorée en rouge vendue sous la dénomination commerciale « FB-400 Orange Red » par la société UK SEUNG CHEMICAL, la résine polyamide colorée en orange vendue sous la dénomination commerciale « Flare 911 Orange 4 » par la société Sterling Industrial Colors.

Les substances fluorescentes sont de préférence présentes dans la composition, à une teneur allant de 0,1 à 20%, de préférence de 0,1 à 15%, de préférence encore de 0,5 à 3% en poids, par rapport au poids total de la composition.

Lorsque les substances fluorescentes organiques sont blanches, on les appelle également des azurants optiques.

L'azurant optique a pour effet d'intensifier l'éclat et aviver les teintes des compositions cosmétiques les comprenant à l'application sur la peau.

Parmi les azurants optiques, on peut plus particulièrement citer les dérivés du stilbène, en particulier les polystyrylstilbènes et les triazinstilbènes, les dérivés coumariniques, en particulier les hydroxycoumarines et les aminocoumarines, les dérivés oxazole, benzoxazole, imidazole, triazole, pyrazoline, les dérivés du pyrène et les dérivés de porphyrine et/ou leurs mélanges.

De tels composés sont par exemple disponibles sous les dénominations commerciales Tinopal SOP□et Uvitex OB auprès de la société CIBA GEIGY.

Les azurants optiques préférentiellement utilisés sont le 4,4'-bis[(4,6-dianilino-1,3,5-triazin-2-yl)amino]stilbène-2,2'-disulfonate de sodium, le 2,5 thiophène di-yl bis(5 ter-butyl-1,3 benzoxazole), le di-styryl-4,4' biphényle sulfonate di-sodique et/ou leurs mélanges.

## Agents additionnels favorisant la coloration naturellement rosée de la peau

On peut citer :

(1) les agents autobronzants tels que décrits précédemment. Ils sont généralement présents dans des proportions

allant de 0,1 à 15% en poids et de préférence de 0,2 à 10% en poids et plus préférentiellement de 1 à 8 % en poids par rapport au poids total de la composition comportant l'acide déhydroascorbique ou l'un de ses polymères et /ou la composition comprenant l'acide ascorbique ou l'un de ses sels ou dérivés.

(2) les agents de coloration directs synthétiques ou naturels, organiques ou minéraux tels que décrits précédemment.

(3) les dérivés indoliques comme les monohydroxyindoles tels que décrits dans le brevet FR2651126 ( ie : 4-, 5-, 6- ou 7-hydroxyindole) ou les di-hydroxyindoles tels que décrits dans le brevet EP-B-0425324 (ie : 5,6-dihydroxyindole, 2-méthyl 5,6-dihydroxyindole, 3-méthyl 5,6-dihydroxyindole, 2,3-diméthyl 5,6-dihydroxyindole).

(4) les colorants obtenus avec les composés comportant au moins un cycle aromatique ayant au moins deux groupes hydroxyle (OH) portés par deux atomes de carbone consécutifs du cycle aromatique et un système catalytique comprenant un premier constituant choisi parmi les sels et oxydes de Mn(II), et/ou Zn(II) et leurs mélanges et un second constituant choisi parmi les hydrogénocarbonates alcalins, les hydrogénocarbonates alcalino-terreux et leurs mélanges, tels que décrits précedemment.

<u>**Charges abrasives ou agents exfoliants**</u>

Comme agents exfoliants utilisables dans des compositions rincées selon l'invention, on peut citer par exemple des particules exfoliantes ou gommantes d'origine minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges.

On peut citer aussi l'Exfogreen® de Solabia (extrait de bambou), des extraits d'akenes de fraises (Akenes de fraise de Greentech), de la poudre de noyau de pêche, la poudre de noyau d'abricot, et enfin dans le domaine des poudres végétales à effet abrasif, citons la poudre de noyaux d'airelles (cranberry).

Comme charges abrasives ou agents exfoliants préférés selon l'invention, on citera la poudre de noyaux de pêche, la poudre de noyaux d'abricot, la poudre de noyaux d'airelles, les extraits d'akènes de fraise, les extraits de bambou.

Le ou les ingrédients additionnels utilisés dans l'une des compositions de soin et/ou de maquillage selon l'invention peuvent représenter de 0,0001 à 20%, de préference de 0,01 à 10% et mieux, de 0,01 à 1 % en poids para rapport au poids total de la composition.

Des exemples concrets, mais nullement limitatifs, illustrant l'invention, vont maintenant être donnés.

<u>**EXEMPLE 1 :**</u>

Les produits sont évalués sur le modèle EPISKIN de peau reconstruite après 18 jours de mise en culture. Les nacelles sont déposées sur papier Wathman. 20 μl de solution à 5% en poids de produit à tester dans un mélange eau 50% et éthanol 50 %. Après 24 heures un lavage est effectué avec 1 ml d'eau contenant du lauryl ether sulfate de sodium à 1 % sous agitation pendant 1 minute. Un second rinçage est effectué avec 1 ml d'eau. Le liquide résiduel est absorbé avec un mouchoir doux. Les mesures sont réalisées avec un spectrocolorimètre Minolta CM-2600d.

<u>Matériel</u>

Spectrocolorimètre Minolta CM2600d
Illuminant: D65
Observateur : 10°
SCE : spéculaire exclus

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

L'écart de couleur est obtenu à l'aide de la formule d'écart de couleur de Hunter dans l'espace colorimétrique L, a, b :

$$(\Delta E)^2 = [(\Delta L)^2 + (\Delta a)^2 + (\Delta b)^2].$$

ΔE traduit l'assombrissement, l'intensité globale de la couleur : plus le ΔE est important plus la couleur s'est assombrie.

$\Delta E^*$ traduit l'assombrissement, l'intensité globale de la couleur : plus le ΔE est important plus la couleur s'est assombrie.

**Résultats après lavage**

| Produit à tester | L* | a* | b* | ΔE*ab |
|---|---|---|---|---|
| Acide déhydroascorbique | 77,7 | 4,8 | 35 | 14,6 |
| Dihydroxyacétone (DHA) | 82,6 | 1,3 | 30,0 | 11,1 |
| Episkin seul | 86,2 | -1,2 | 24,7 | |
| L = intensité, a composante rouge, b composante jaune | | | | |

On observe qu'à concentration égale de 5% en poids, l'acide deshydroascorbique permet d'obtenir une nuance plus intense à dominante rouge plus marquée que celle obtenue avec la DHA.

**EXEMPLE 2 : COLORATIONS A PARTIR DE LA VITAMINE C**

Exemple 2a : (oxydation chimique)

On effectue une oxydation de la vitamine C sous forme d'ascorbate de sodium en mélangeant 5,7 mmoles de peroxyde d'hydrogène à 5,7 mmoles d'ascorbate de sodium. On laisse réagir le mélange pendant 2 heures à 30 °C.

Exemple 2b : (oxydation enzymatique par une enzyme libre)

On effectue une oxydation de l'ascorbate de sodium par réaction enzymatique avec l'ascorbate oxydase en mélangeant extemporanément

(1) 5 µl d'une solution d'ascorbate oxydase à 5 unités/ml dans un tampon de phosphate à 0,1 M et pH 7,5 avec
(2) 20 µl de solution d'ascorbate de sodium 10 mM dans un tampon phosphate à 0,1 M et pH 7,5

Exemple 2c : (oxydation enzymatique par une enzyme immobilisée)

On effectue une oxydation de l'ascorbate de sodium en effectuant une percolation de 1 ml d'une solution à 5% d'ascorbate de sodium ans un tampon phosphate à 0,1 M et pH 7,5 sur de l'ascorbate oxydase immobilisée sur une résine époxy du type EUPERGIT C.

On applique pour chacune des solutions résultantes, 25 µl de solution sur BLENDERM qui est du stratum prélevé au moyen d'un polymère du type 3M Scotch. On mesure la couleur obtenue au boout de 4 heures avec le colorimètre Minolta. On évalue l'évolution de la coloration par rapport à des échantillons témoins non traités.

Matériel

Spectrocolorimètre Minolta CM2600d
Illuminant: D65
Observateur : 10°
SCE : spéculaire exclus

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

L'écart de couleur est obtenu à l'aide de la formule d'écart de couleur de Hunter dans l'espace colorimétrique L, a, b :

$$(\Delta E)^2 = [(\Delta L)^2 + (\Delta a)^2 + (\Delta b)^2].$$

ΔE traduit l'assombrissement, l'intensité globale de la couleur : plus le ΔE est important plus la couleur s'est assombrie.

ΔE* traduit l'assombrissement, l'intensité globale de la couleur : plus le ΔE est important plus la couleur s'est assombrie.

**Résultats**

| Produit à tester | L* | a* | b* | ΔE* |
|---|---|---|---|---|
| Ex 2a Oxydation de la vitamine C chimique | 84,26 | -1,78 | 21,7 | 17,0 |
| Ex2b Oxydation de la vitamine C avec enzyme libre | 87,16 | -0,26 | 10,4 | 5,4 |
| Ex 2 c Oxydation de la vitamine C avec enzyme immobilisée | 85,61 | 1,84 | 21,27 | 16,5 |

**EXEMPLE 3 : Crème de soin (émulsion huile dans eau)**

| | | |
|---|---|---|
| A- | Glyceryl stearate (et) PEG-1 00 stearate : | 2.00 % |
| | Dimyristyl tartrate (et) cetearyl alcohol (et) C12-15 pareth-7 (et) PP%-25 laureth-25 | 1.50 % |
| | Cyclohexasiloxane : | 10.00 % |
| | Alcool Stearylique : | 1.00 % |
| B- | Eau : | 80.75% |
| | Phenoxyethanol : | 1.00 % |
| | Pentasodium ethylene diamine tetramethylene phosphate: | 0.05 % |
| | Ammonium Polyacryldimethyltauramide: | 0.40 % |
| | Xanthan gum : | 0.20 % |
| C- | Dehydroascorbic Acid : | 3.00 % |

Mode opératoire :

- Chauffer la phase B à environ 75°C et y incorporer l'Ammonium Polyacryldimethyltauramide; agiter jusqu'à obtention d'un gel homogène.
- chauffer la phase A à environ 75°C.
- réaliser l'émulsion en incorporant la phase A dans la phase B.
- à 40-45°C, incorporer la phase C et maintenir l'agitation jusqu'à refroidissement complet.

**EXEMPLE 4 : Gel-crème**

| | | |
|---|---|---|
| A- | Cyclomethicone | 5 % |
| | Hydrogenated polyisobutene | 5 % |
| | Xanthan gum | 0.4 % |
| | Acrylates / $C_{10}$-$C_{30}$ alkyl acrylate crosspolymer | 0.25 % |
| | Conservateurs | 1 % |
| B- | Eau | 77.65% |
| | Glycerine | 5 % |
| | Dimethicone PEG-7 phosphate | 2 % |

(suite)

| | |
|---|---|
| Carbomer | 0.4 % |
| Sodium hydroxide | 0.3 % |
| Dehydroascorbic Acid Dimer | 3 % |

Mode opératoire : les phases A et B sont homogénéisées ; ambiante, puis l'émulsion est réalisée en introduisant la phase A da sous forte agitation.

**EXEMPLE 5 : Formulation à deux composants**

Composant (1) : Emulsion huile dans eau avec acide ascorbique

| | | |
|---|---|---|
| A- | Stearate de polyethylene glycol (20 OE) | 0,8% |
| | Mono/distearate de glyceryle / stearate de polyethylene glycol (100 OE) | 2% |
| | Alcool cetylique | 0,7% |
| | Alcool stearylique | 0,7% |
| | Acide stearique | 1% |
| | Stearate d'iso-cetyle | 6% |
| | Cire d'abeille | 1% |
| | Conservateurs | 0,6 |
| B- | Glycérine | 7% |
| | Eau | QSP% |
| | Triethanolamine | 0,23% |
| C- | Cyclopenta dimethylsiloxane | 10% |
| D- | Acrylamide/Sodium Acryloyldimethyltaurate Copolymer (And) Isohexadecane (And) Polysorbate 80 | 1,6% |
| E- | Acide Ascorbique | 5% |

Mode opératoire :

- Chauffer les phases A et B à environ 75°C
- Réaliser l'émulsion en introduisant la phase A dans la phase B sous forte agitation
- à 40-45°C, incorporer la phase C puis D et maintenir l'agitation jusqu'à refroidissement complet.
- Incorporer alors la phase E à froid

Composant (2) : Gel aqueux contenant de l'ascorbate oxydase

| | | |
|---|---|---|
| A- | Eau | QSP |
| | Glycerol | 7% |
| | 1,3-butylene glycol | 5% |
| B- | Laponite synthetique (silicate mixte magnesium/lithium/sodium) | 3% |
| C- | Alcool ethylique | 10% |

(suite)

| | |
|---|---|
| Octane-1,2 diol | 0,4% |

Mode opératoire :

- Chauffer la phase A à 65°C
- Ajouter la phase B puis laisser revenir à température ambiante.
- Incorporer la phase C

Les deux composants sont conditionnés dans un packaging double corps avec pompe de distribution séparée permettant le mélange à l'application.

**<u>EXEMPLE 6 :</u> formulation à trois composants**

<u>Composant (1) : Emulsion huile dans eau avec acide ascorbique</u>

| | | |
|---|---|---|
| A- | Stearate de polyethylene glycol (20 OE) | 0,8% |
| | Mono/distearate de glyceryle / stearate de polyethylene glycol (100 OE) | 2% |
| | Alcool cetylique | 0,7% |
| | Alcool stearylique | 0,7% |
| | Acide stearique | 1% |
| | Stearate d'iso-cetyle | 6% |
| | Cire d'abeille | 1 % |
| | Conservateurs | 0,6 |
| B- | glycerol | 7% |
| | Eau | QSP% |
| | Triethanolamine | 0,23% |
| C- | Cyclopenta dimethylsiloxane | 10% |
| D- | Acrylamide/Sodium Acryloyldimethyltaurate Copolymer (And) Isohexadecane (And) Polysorbate 80 | 1,6% |
| E- | Acide Ascorbique | 5% |

Mode opératoire :

- Chauffer les phases A et B à environ 75°C
- Réaliser l'émulsion en introduisant la phase A dans la phase B sous forte agitation
- à 40-45°C, incorporer la phase C puis D et maintenir l'agitation jusqu'à refroidissement complet.
- Incorporer alors la phase E à froid

<u>Composant (2) : Emulsion eau dans huile contenant de l'acide dehydroascorbique</u>

| | | |
|---|---|---|
| A- | Mono-stearate de glyceryle / stearate de polyethylene glycol (100 OE) | 2% |
| | Arachydyl polyglucoside / alcools arachidique, behenique | 3% |

(suite)

| | | |
|---|---|---|
| | Iso-hexadecane | 4% |
| | Poly dimethylsiloxane | 1% |
| | Benzoate d'alcools $C_{12}/C_{15}$ | 4% |
| | Citrate de tri-alkyle | 3% |
| | Conservateurs | 0,5% |
| B - | Acide ethylene diamine tetracetique | 0,1% |
| | Glycerol | 4% |
| | Propylene glycol | 6% |
| | Eau | 10% |
| | Conservateurs | 0,2% |
| C- | Polyacrylamide /C13-14 Isoparaffin / Laureth-7 | 1% |
| D- | Mélange de tocopherols naturels dans huile de soja (50/50) | 0,1% |
| E- | Dehydroascorbic Acid Dimer | 5% |
| | Eau desionisee microbiologiquement propre (qs) | qsp |
| F- | Alcool ethylique | 5% |

Mode opératoire :

- Chauffer les phases A et B à environ 75°C
- Réaliser l'émulsion en introduisant la phase A dans la phase B sous forte agitation et y incorporer la phase D; agiter jusqu'à obtention d'un gel homogène.
- à 40-45°C, incorporer les phases D puis E et maintenir l'agitation jusqu'à refroidissement complet.
- Incorporer alors la phase E à froid

<u>Composant (3) : Gel aqueux contenant de l'ascorbate oxydase</u>

| | | |
|---|---|---|
| A- | Eau | qsp |
| | Glycerol | 7% |
| | 1,3-Butylene glycol | 5% |
| B- | Laponite synthetique (silicate mixte magnesium/lithium/sodium) | 3% |
| C- | Alcool ethylique | 10% |
| | Octane-1,2 diol | 0,4% |

Mode opératoire :

- Chauffer la phase A à 65°C
- Ajouter la phase B puis laisser revenir à température ambiante.
- Incorporer la phase C

**Revendications**

1. **Utilisation cosmétique comme agent de coloration artificielle de la peau dans une composition comprenant un milieu physiologiquement acceptable,** de l'acide déhydroascorbique et/ou l'un de ses dérivés monomères de formule (I) suivante et/ou l'un de ses isomères de formule (I') suivante:

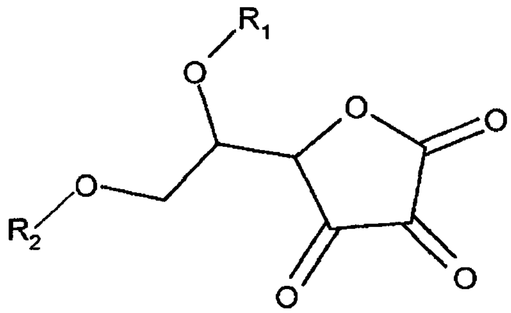

(I)

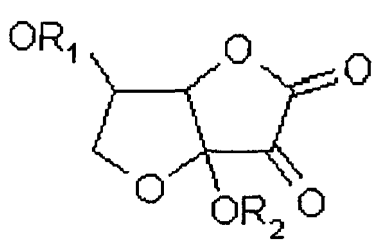

(I')

dans lesquelles $OR_1$, $OR_2$, identiques ou différents, désignent OH ; un radical alcoxy en $C_1$-$C_{30}$ et plus préférentiellement en $C_1$-$C_{18}$, linéaire ou ramifié, saturé ou insaturé ; un glycoside et plus préférentiellement glucose ; un ester d'un acide carboxylique aliphatique en $C_1$-$C_{30}$ (de préférence en $C_1$-$C_{18}$), linéaire ou ramifié, saturé ou insaturé, pouvant être substitué par un groupe aryle ou un hétérocycle ; un ester d'acide carboxylique arylique, hétérocyclique pouvant être substitué par au moins un radical alkyle en $C_1$-$C_{30}$ (plus préférentiellement en $C_1$-$C_{18}$), linéaire ou ramifié, saturé ou insaturé ; un groupe phosphate ; un groupe sulfate,
**et/ou l'un des composés** dimères de formule (II) suivante :

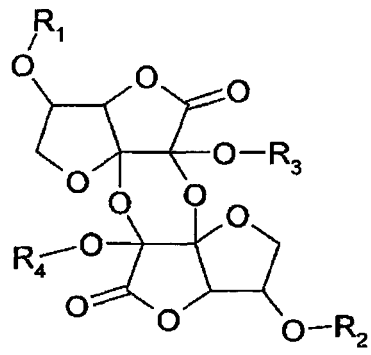

(II)

**dans laquelle $OR_1$, $OR_2$, $OR_3$, $OR_4$, identiques ou différents, identiques ou différents, désignent OH; un radical alcoxy en $C_1$-$C_{30}$ et plus préférentiellement en $C_1$-$C_{18}$, linéaire ou ramifié, saturé ou insaturé ; un glycoside et plus préférentiellement glucose ; un ester d'un acide carboxylique aliphatique en $C_1$-$C_{30}$ (de préférence en $C_1$-$C_{18}$), linéaire ou ramifié, saturé ou insaturé, pouvant être sustitué par un groupe aryle ou un hétérocycle; un ester d'acide carboxylique arylique, hétérocyclique pouvant être substitué par au moins un radical alkyle en $C_1$-$C_{30}$ ( plus préférentiellement en $C_1$-$C_{18}$), linéaire ou ramifié, saturé ou insaturé ; un groupe phosphate ; un groupe sulfate.**

2. Utilisation selon la revendication 1, où le composé de formule (I) est l'acide dehydroascorbique de structure :

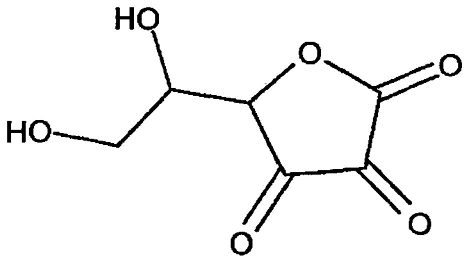

3. Utilisation selon la revendication 1, où le composé de formule (I') est la 3a,6-dihydroxy-tetrahydro-furo[3,2-b]furan-2,3-dione a pour structure :

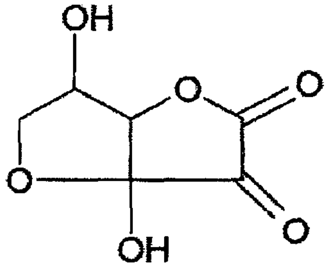

4. Utilisation selon la revendication **1**, où le dimère est le composé de structure suivante :

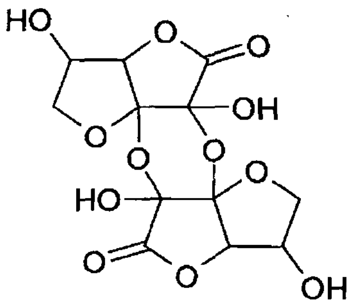

5. Procédé de coloration artificielle de la peau et plus particulièrement de bronzage artificiel de la peau, **caractérisé en ce qu'**il consiste à appliquer sur celle-ci une quantité efficace d'une composition cosmétique comprenant dans un milieu physiquement acceptable au moins l'acide déhydroascorbique et/ou l'un de ses dérivés monomères de formule (I) et/ou l'un de ses isomères de formule (I') et/ou l'un de ses **dimères de formule (II)** tels que définis dans les revendications 1 à 4.

6. Procédé **selon la revendication 5,** où l'acide déhydroascorbique est formé « in situ » à partir de l'acide ascorbique ou l'un de ses dérivés ou l'un de ses sels par oxydation chimique et/ou par oxydation enzymatique.

7. **Procédé** selon la revendication **6**, d'un agent à deux composants comprenant

a) un premier composant (B) contenant dans un milieu physiologiquement acceptable au moins l'acide ascorbique ou l'un de ses dérivés ou sels ;
b) un deuxième composant (C) contenant dans un milieu physiologiquement acceptable au moins un agent oxydant chimique et/ou un agent oxydant enzymatique ; les composants (B) et (C) étant stockés séparément, pour la coloration artificielle de la peau en particulier comme agent autobronzant de la peau.

8. **Procédé** selon la revendication **7**, d'un agent à trois composants comprenant comprenant :

a) un premier composant (A) contenant dans un milieu physiologiquement acceptable au moins l'acide déhydroascorbique et/ou l'un de ses dérivés monomères de formule (I) et/ou l'un de ses isomères de formule (I') et/ou l'un de ses **dimères de formule (II)** ;
b) un deuxième composant (B) contenant dans un milieu physiologiquement acceptable au moins l'acide ascorbique ou l'un de ses dérivés ou sels ;
c) un troisième composant (C) contenant dans un milieu physiologiquement acceptable au moins un agent oxydant chimique et/ou un agent oxydant enzymatique ; les composants (A) et (B) pouvant être dans la même composition ou stockés séparément et le composant (C) étant stocké séparément de (A) et de (B).

**9. Procédé** selon l'une quelconque des revendications **7 et 8**, où

(i) l'agent oxydant chimique est choisi parmi le peroxyde d'hydrogène, le peroxyde d'urée, les bromates de métaux alcalins, les persels tels que les perborates et persulfates, les peracides ou leurs mélanges.
(ii) l'agent oxydant enzymatique est choisi parmi les enzymes oxydases.

**10. Procédé** selon l'une quelconque des revendications **5 à 9**, où l'on associe l'acide déshydroascorbique et/ou l'un de ses dérivés monomères de formule (I) et/ou l'un de ses isomères de formule (I') et/ou l'un de ses **dimères de formule (II)** avec l'acide ascorbique ou l'un de ses dérivés ou l'un de ses sels.

**11. Procédé** selon l'une quelconque des revendications **5** à **10**, **caractérisés par le fait que** la composition comprenant l'acide déhydroascorbique et/ou l'un de ses dérivés monomères de formule (I) et/ou l'un de ses isomères de formule (I') et/ou l'un de ses **dimères de formule (II)** et /ou la composition comprenant l'acide ascorbique ou l'un de ses sels ou dérivés contient en plus un ou plusieurs stabilisants, et/ou un agent photoprotecteur et/ou un agent mouillant et/ou pénétrant et/ou au moins un colorant additionnel.

## Patentansprüche

**1.** Kosmetische Verwendung von Dehydroascorbinsäure und/oder einem monomeren Derivat davon der folgenden Formel (I) und/oder einem Isomer davon der folgenden Formel (I'):

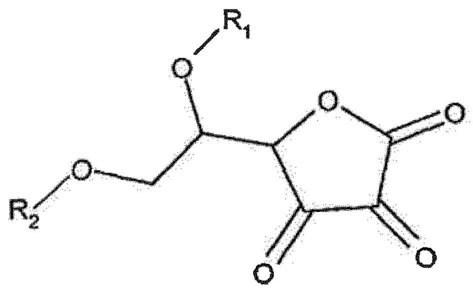

(I)

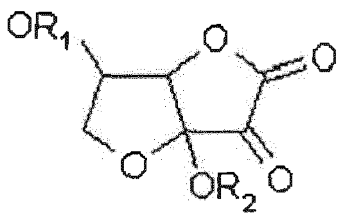

(I')

worin $OR_1$ und $OR_2$ gleich oder verschieden sind und für OH; einen linearen oder verzweigten, gesättigten oder ungesättigten $C_1$-$C_{30}$- und weiter bevorzugt $C_1$-$C_{18}$-Alkoxyrest; ein Glycosid und weiter bevorzugt Glucose; einen linearen oder verzweigten, gesättigten oder ungesättigten aliphatischen $C_1$-$C_{30}$-Carbonsäureester (vorzugsweise $C_1$-$C_{18}$-Carbonsäureester), der durch eine Arylgruppe oder einen Heterocyclus substituiert sein kann; einen Aryl- oder Heterocyclylcarbonsäureester, der durch mindestens einen linearen oder verzweigten, gesättigten oder ungesättigten $C_1$-$C_{30}$-Alkylrest (weiter bevorzugt $C_1$-$C_{18}$-Alkylrest) substituiert sein kann; eine Phosphatgruppe oder eine

Sulfatgruppe stehen,
und/oder einer der dimeren Verbindungen der folgenden Formel (II):

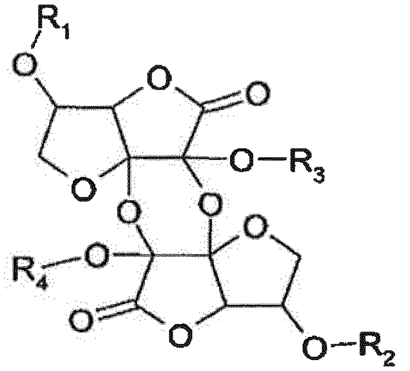

(II)

worin $OR_1$, $OR_2$, $OR_3$ und $OR_4$ gleich oder verschieden sind und für OH; einen linearen oder verzweigten, gesättigten oder ungesättigten $C_1$-$C_{30}$- und weiter bevorzugt $C_1$-$C_{18}$-Alkoxyrest; ein Glycosid und weiter bevorzugt Glucose; einen linearen oder verzweigten, gesättigten oder ungesättigten aliphatischen $C_1$-$C_{30}$-Carbonsäureester (vorzugsweise $C_1$-$C_{18}$-Carbonsäureester), der durch eine Arylgruppe oder einen Heterocyclus substituiert sein kann; einen Aryl- oder Heterocyclylcarbonsäureester, der durch mindestens einen linearen oder verzweigten, gesättigten oder ungesättigten $C_1$-$C_{30}$-Alkylrest (weiter bevorzugt $C_1$-$C_{18}$-Alkylrest) substituiert sein kann; eine Phosphatgruppe oder eine Sulfatgruppe stehen,
als Mittel zur künstlichen Färbung der Haut in einer Zusammensetzung, die ein physiologisch unbedenkliches Medium umfasst.

**2.** Verwendung nach Anspruch 1, wobei es sich bei der Verbindung der Formel (I) um Dehydroascorbinsäure der Struktur:

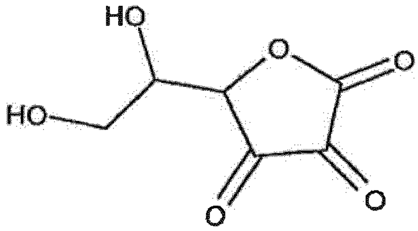

handelt.

**3.** Verwendung nach Anspruch 1, wobei es sich bei der Verbindung der Formel (I') um 3a,6-Dihydroxytetrahgydrofuro[3,2-b]furan-2,3-dion mit der Struktur:

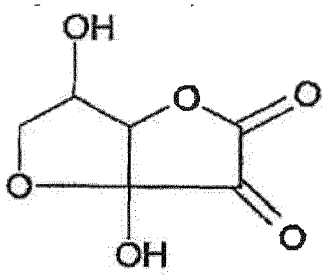

handelt.

**4.** Verwendung nach Anspruch 1, wobei es sich bei dem Dimer um die Verbindung der folgenden Struktur:

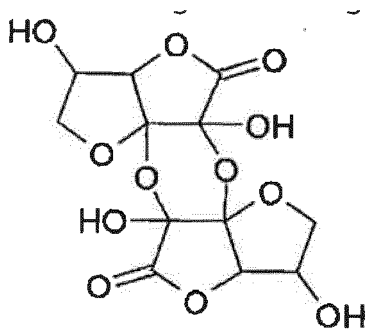

handelt.

**5.** Verfahren zur künstlichen Färbung der Haut und insbesondere zur künstlichen Bräunung der Haut, **dadurch gekennzeichnet, dass** es daraus besteht, dass man auf die Haut eine wirksame Menge einer kosmetischen Zusammensetzung, die in einem physiologisch unbedenklichen Medium mindestens Dehydroascorbinsäure und/oder ein monomeres Derivat davon der Formel (I) und/oder ein Isomer davon der Formel (I') und/oder ein Dimer davon der Formel (II) gemäß den Ansprüchen 1 bis 4 umfasst, aufbringt.

**6.** Verfahren nach Anspruch 5, bei dem die Dehydroascorbinsäure in situ aus Ascorbinsäure oder einem Derivat davon oder einem Salz davon durch chemische Oxidation und/oder durch enzymatische Oxidation gebildet wird.

**7.** Verfahren nach Anspruch 6 eines zweikomponentigen Mittels, das

a) eine erste Komponente (B), die in einem physiologisch unbedenklichen Medium mindestens Ascorbinsäure oder ein Derivat oder Salz davon enthält;
b) eine zweite Komponente (C), die in einem physiologisch unbedenklichen Medium mindestens ein chemisches Oxidationsmittel und/oder ein enzymatisches Oxidationsmittel enthält,
umfasst; wobei die Komponenten (B) und (C) separat aufbewahrt werden, zur künstlichen Färbung der Haut und insbesondere als Selbstbräunungsmittel für die Haut.

**8.** Verfahren nach Anspruch 7 eines dreikomponentigen Mittels, das

a) eine erste Komponente (A), die in einem physiologisch unbedenklichen Medium mindestens Dehydroascorbinsäure und/oder ein monomeres Derivat davon der Formel (I) und/oder ein Isomer davon der Formel (I') und/oder ein Dimer davon der Formel (II) enthält;
b) eine zweite Komponente (B), die in einem physiologisch unbedenklichen Medium Ascorbinsäure oder ein Derivat oder Salz davon enthält;
c) eine dritte Komponente (C), die in einem physiologisch unbedenklichen Medium mindestens ein chemisches Oxidationsmittel und/oder ein enzymatisches Oxidationsmittel enthält,
umfasst; wobei die Komponenten (A) und (B) können separat aufbewahrt werden in derselben Zusammensetzung vorliegen oder und die Komponente (C) separat von (A) und (B) aufbewahrt wird.

**9.** Verfahren nach einem der Ansprüche 7 und 8, bei dem

(i) das chemische Oxidationsmittel aus Wasserstoffperoxid, Harnstoffperoxid, Alkalimetallbromaten, Persalzen wie Perboraten und Persulfaten, Persäuren oder Mischungen davon ausgewählt wird;
ii) das enzymatische Oxidationsmittel aus Oxydase-Enzymen ausgewählt wird.

**10.** Verfahren nach einem der Ansprüche 5 bis 9, bei dem man Dehydroascorbinsäure und/oder ein monomeres Derivat davon der Formel (I) und/oder ein Isomer davon der Formel (I') und/oder ein Dimer davon der Formel (II) mit Ascorbinsäure oder einem Derivat davon oder einem Salz davon vereinigt.

**11.** Verfahren nach einem der Ansprüche 5 bis 10, **dadurch gekennzeichnet, dass** die Zusammensetzung, die Dehydroascorbinsäure und/oder ein monomeres Derivat davon der Formel (I) und/oder ein Isomer davon der Formel (I') und/oder ein Dimer davon der Formel (II) umfasst, und/oder die Zusammensetzung, die Ascorbinsäure oder ein Salz oder Derivat davon umfasst, außerdem einen oder mehrere zusätzliche Stabilisatoren und/oder ein Lichtschutzmittel und/oder ein Netzmittel und/oder ein Penetriermittel und/oder mindestens ein zusätzliches Farbmittel enthält.

## Claims

1. Cosmetic use as an agent for artificially colouring the skin in a composition comprising a physiologically acceptable medium, of dehydroascorbic acid and/or one of the monomeric derivatives thereof of formula (I) below and/or one of the isomers thereof of formula (I') below:

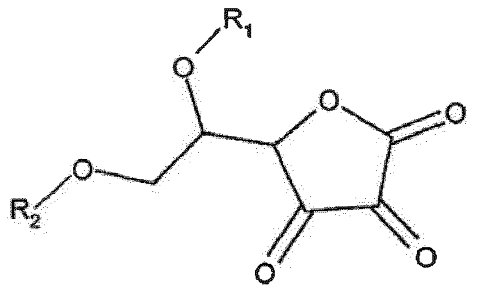

(I)

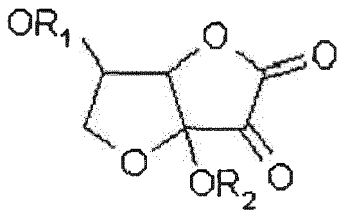

(I')

in which $OR_1$ and $OR_2$, which may be identical or different, denote OH; a linear or branched, saturated or unsaturated $C_1$-$C_{30}$ and more preferentially $C_1$-$C_{18}$ alkoxy radical; a glycoside and more preferentially glucose; a linear or branched, saturated or unsaturated $C_1$-$C_{30}$ (preferably $C_1$-$C_{18}$) aliphatic carboxylic acid ester, which may be substituted with an aryl group or a heterocycle; an aryl or heterocyclic carboxylic acid ester which may be substituted with at least one linear or branched, saturated or unsaturated $C_1$-$C_{30}$ (more preferentially $C_1$-$C_{18}$) alkyl radical; a phosphate group; a sulfate group, and/or one of the dimeric compounds of formula (II) below:

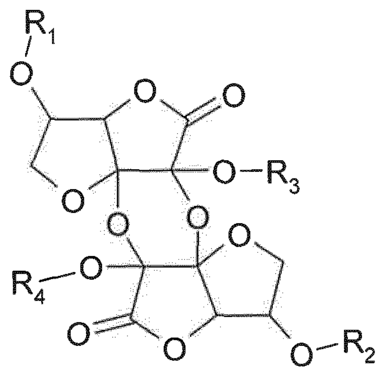

(II)

in which $OR_1$, $OR_2$, $OR_3$ and $OR_4$, which may be identical or different, denote OH; a linear or branched, saturated or unsaturated $C_1$-$C_{30}$ and more preferentially $C_1$-$C_{18}$ alkoxy radical; a glycoside and more preferentially glucose; a linear or branched, saturated or unsaturated $C_1$-$C_{30}$ (preferably $C_1$-$C_{18}$) aliphatic carboxylic acid ester, which may be substituted with an aryl group or a heterocycle; an aryl or heterocyclic carboxylic acid ester which may be substituted with at least one linear or branched, saturated or unsaturated $C_1$-$C_{30}$ (more preferentially $C_1$-$C_{18}$) alkyl radical; a phosphate group; a sulfate group.

2. Use according to Claim 1, in which the compound of formula (I) is dehydroascorbic acid of structure:

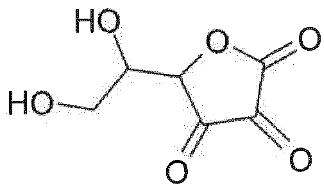

3. Use according to Claim 1, in which the compound of formula (I') is 3a,6-dihydroxytetrahydrofuro[3,2-b]furan-2,3-dione of structure:

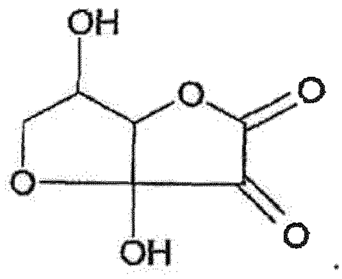

4. Use according to Claim 1, in which the dimer is the compound having the following structure:

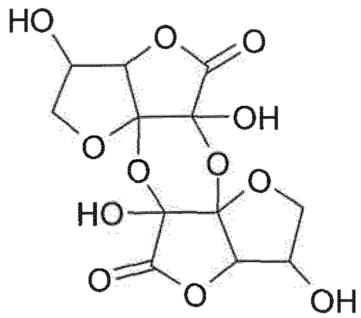

5. Process for artificially colouring the skin and more particularly for artificially tanning the skin, **characterized in that** it consists in applying to the skin an effective amount of a cosmetic composition comprising, in a physiologically acceptable medium, at least dehydroascorbic acid and/or one of the monomeric derivatives thereof of formula (I) and/or one of the isomers thereof of formula (I') and/or one of the dimers thereof of formula (II) as defined in Claims 1 to 4.

6. Process according to Claim 5, in which the dehydroascorbic acid is formed "in situ" from ascorbic acid or a derivative or salt thereof via chemical oxidation and/or via enzymatic oxidation.

7. Process according to Claim 6, of a two-component agent comprising:

a) a first component (B) containing, in a physiologically acceptable medium, at least ascorbic acid or a derivative or salt thereof;
b) a second component (C) containing, in a physiologically acceptable medium, at least one chemical oxidizing agent and/or one enzymatic oxidizing agent; components (B) and (C) being stored separately, for the artificial colouring of the skin, in particular as a skin self-tanning agent.

8. Process according to Claim 7, of a three-component agent comprising:

a) a first component (A) containing, in a physiologically acceptable medium, at least dehydroascorbic acid and/or one of the monomeric derivatives thereof of formula (I) and/or one of the isomers thereof of formula (I') and/or one of the dimers thereof of formula (II);

b) a second component (B) containing, in a physiologically acceptable medium, at least ascorbic acid or a derivative or salt thereof;
c) a third component (C) containing, in a physiologically acceptable medium, at least one chemical oxidizing agent and/or an enzymatic oxidizing agent; components (A) and (B) possibly being in the same composition or stored separately and component (C) being stored separately from (A) and from (B).

**9.** Process according to either of Claims 7 and 8, in which

(i) the chemical oxidizing agent is chosen from hydrogen peroxide, urea peroxide, alkali metal bromates, persalts such as perborates and persulfates, and peracids, or mixtures thereof;
(ii) the enzymatic oxidizing agent is chosen from oxidase enzymes.

**10.** Process according to any one of Claims 5 to 9, in which the dehydroascorbic acid and/or one of the monomeric derivatives thereof of formula (I) and/or one of the isomers thereof of formula (I') and/or one of the dimers thereof of formula (II) is combined with ascorbic acid or a derivative or salt thereof.

**11.** Process according to any one of Claims 5 to 10, **characterized in that** the composition comprising dehydroascorbic acid and/or one of the monomeric derivatives thereof of formula (I) and/or one of the isomers thereof of formula (I') and/or one of the dimers thereof of formula (II) and/or the composition comprising ascorbic acid or a salt or derivative thereof also contains one or more additional stabilizers, and/or a photoprotective agent and/or a wetting agent and/or penetrant and/or at least one additional colorant.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2005039510 A
- DE 19745354
- JP 2295912 A
- EP 1316302 A
- WO 9745517 A
- WO 1481677 A
- US 4234435 A
- US 4708753 A
- US 5129972 A
- US 4931110 A
- GB 2156799 A
- US 4919179 A
- EP 1374849 A
- US 5624663 A
- EP 669323 A
- US 2463264 A
- US 5237071 A
- US 5166355 A
- GB 2303549 A
- DE 19726184
- EP 893119 A
- EP 0832642 A
- EP 1027883 A
- EP 1300137 A
- DE 10162844
- WO 9304665 A
- DE 19855649
- EP 0967200 A
- DE 19746654
- DE 19755649
- EP 1008586 A
- EP 1133980 A
- EP 133981 A
- US 6225467 B
- WO 2004085412 A
- WO 06035000 A
- WO 06034982 A
- WO 06034991 A
- WO 06035007 A
- WO 2006034992 A
- WO 2006034985 A
- EP 966953 A
- EP 1172091 A
- FR 2651126
- EP 0425324 B
- WO 02051828 A
- EP 0852949 A
- EP 0796861 A
- EP 0218200 A
- EP 0899330 A
- EP 1484052 A
- WO 0247650 A
- FR 2802425
- FR 2810548
- FR 2796278
- FR 2802420
- FR 2812544 A
- FR 2814950 A
- WO 0194381 A
- FR 2877220
- WO 03030937 A
- WO 03068753 A
- WO 0044384 A
- EP 1038519 A
- EP 761204 A
- DE 10324567
- WO 9318743 A
- FR 0108283
- EP 1529523 A
- EP 1133979 A
- EP 1129694 A
- EP 1562562 A
- EP 1151742 A
- FR 2869796
- US 5538793 A
- EP 1579849 A

### Littérature non-brevet citée dans la description

- **LEE, M.H. ; DAWSON , C.R.** *Methods Enzymol.,* 1979, vol. 62, 30-39
- Symetrical Triazine Derivatives. IP.COM Journal. IP.COM, 20 Septembre 2004
- *Cosmetics & Toiletries,* Février 1990, vol. 105, 53-64